# EUROPEAN PATENT APPLICATION

(11) **EP 4 242 222 A1**
(43) Date of publication of application: **13.09.2023**
(21) Application number: 21834711.0
(22) Date of filing: 03.11.2021
(51) Int. Cl.: C07K 14/005, A61K 39/12, A61P 31/14

(54) **CHIMERIC PROTEIN COMPRISING THE RECEPTOR BINDING DOMAIN OF THE CORONAVIRUS SPIKE PROTEIN AND COMPOSITIONS COMPRISING THEM**

(30) Priority: 04.11.2020 CU 20200081
(71) Applicant: Centro de Ingeniería Genética y Biotecnología, La Habana 11600 (CU)
(72) Inventor: CHINEA SANTIAGO, Glay, Playa, La Habana 11600 (CU); MARTÍN DUNN, Alejandro, Miguel, Playa, La Habana 11600 (CU); GONZÁLEZ ROCHE, Diamilé, Playa, La Habana 11600 (CU); LIMONTA FERNÁNDEZ, Miladys, Playa, La Habana 11300 (CU); IGLESIAS PÉREZ, Enrique, Habana del Este, La Habana 11700 (CU); BEQUET ROMERO, Mónica, Plaza de la Revolucíon, La Habana 10400 (CU); SANTANA MILIAN, Héctor, Playa, La Habana 11600 (CU); MARQUEZ PERERA, Gabriel, J., Marianao, La Habana 11500 (CU); MUSACCHIO LASA, Alexis, Mariel, Artemisa 32100 (CU); CABRALES RICO, Ania, Boyeros, La Habana 10800 (CU); GUILLEN NIETO, Gerardo, Enrique, Playa, La Habana 11600 (CU); AYALA ÁVILA, Marta, Playa, La Habana 11600 (CU); PIMENTEL VAZQUEZ, Eulogio, Playa, La Habana 11300 (CU); ROJAS DORANTES, Gertrudis, 10 de Octubre, La Habana 10500 (CU); HUERTA GALINDO, Vivian, Playa, La Habana 11600 (CU)
(74) Representative: V.O.
(86) International application number: PCT/CU2021/050010
(87) International publication number: WO 2022/096039

(57) **Abstract**

The present invention provides a chimeric protein that has a modular structure and comprises a receptor binding domain (RBD) from the spike protein (S) of coronaviruses, a segment able to bind the core antigen from the Hepatitis B Virus (HBcAg), a segment including six consecutive histidine residues (HHHHHH), and two spacer segments. In this chimeric protein the aforementioned segments are arranged in a specific order, and the protein is able to form hybrid nanoparticles with HBcAg. The chimeric protein is part of vaccine compositions used for the prevention of coronavirus infections. Therefore, the invention discloses a method for the prevention of coronavirus infections, whereby a vaccine composition comprising said chimeric protein is administered.

## Description

### Field of the invention

The present invention pertains to the fields of biopharmaceutical industry and biomedicine, and in particular, the field of vaccines. Especially, the invention pertains to vaccines against coronaviruses, which are infectious agents that cause different diseases, including Coronavirus Disease 2019 (COVID-19). The invention discloses the design and synthesis of chimeric antigens and vaccine compositions thereof that induce a robust immune response and can be administered by different routes.

### State of the art

*Coronaviridae* is a virus family whose members, the so-called coronaviruses, cause a wide-ranging spectrum of disease in animals and humans. Up to year 2003 coronaviruses did not attract much research interest; a situation that changed dramatically with the appearance, that year, of zoonotic SARS-CoV (the causative coronavirus of Severe Acute Respiratory Syndrome) and the emergence, a decade later, of MERS-CoV (causative coronavirus of the Middle East Respiratory Syndrome). These coronaviruses have been confirmed as an important cause of severe respiratory illnesses, and have been responsible for two epidemics totaling close to 10,000 cases. A new coronavirus, SARS-CoV-2, has recently been reported and found to be the infectious agent responsible for the outbreak of COVID-19 that later became pandemic, as declared by the World Health Organization (WHO). This pandemic has swiftly spread worldwide with millions of SARS-CoV-2-positive cases and hundreds of thousands of deaths up to October 2020.

It is known that in COVID-19 convalescents most of the neutralizing response against SARS-CoV-2, as well as a significant portion of the cellular response thereto, is targeted to the Spike protein (S) (Cao Y, *et al.* 2020 May; Brouwer PJM, et al. Science (80- ) 2020; 369(6504): 643-50). Thus, this protein constitutes a prime candidate for inclusion into a vaccine preparation against this pathogen, as confirmed by previous literature on other coronaviruses such as SARS and MERS (Zhou Y, Jiang S, Du L. Expert Rev Vaccines. 2018; 17(8): 677-86; Wang N, Shang J, Jiang S, Du L. Front Microbiol. 2020; 11; Jiang S, He Y, Liu S. Emerg Infect Dis. 2005; 11(7): 1016-20). The existing literature on other pathogenic coronaviruses, however, also contains reports on the potential implication of the S protein in instances of pathological immunoamplification, both in animal models and in clinical settings (Lambert P, et al. Vaccine. 2020 Jun;38(31):4783-91). Thus, the use of protein S in an experimental vaccine is not completely devoid of risk. Additionally, the heterologous expression of protein S, should a subunit vaccine strategy be pursued, represents a formidable challenge: it is a large protein (141 kDa) whose active form is a trimer in prefusion conformation, with 12 to 22 N-glycosylation sites and a variable number of O-glycosylation sites (Zhang Y, et al. bioRxiv. 2020; Shajahan A, Supekar NT, Gleinich AS, Azadi P. Glycobiology, 2020; 1-8), folded with the help of 13 intramolecular disulfide bonds (Kumar S, Maurya VK, Prasad AK, Bhatt MLB, Saxena SK. Virus Disease. 2020; Wrapp D, et al. Science (80- ) 2020 Feb 19;2011(2865)). As a matter of fact, there are no reliable reports of the heterologous expression of protein S in active, prefusion, trimeric conformation in microbial systems (yeast, fungi or bacteria). Protein S is responsible for the interaction of SARS-CoV-2 with ACE2, its receptor in human cells (Wrapp D, et al. Science (80- ). 2020 Feb 19;2011(2865), Wang Q, et al. Cell. 2020; 894-904). This interaction involves a structurally separate domain of protein S, known as the Receptor Binding Domain, or RBD, which extends approximately from cysteine 336 to cysteine 525 and has a molecular weight around 25 kDa. Although RBD does move relative to the remainder of protein S (it is found in two alternative conformations, "open" or "up, and "closed" or "down", which play a central role in the interaction of the S trimer with its receptor) (Ke Z, et al. bioRxiv 2020), the structure of the RBD itself is rigidly constrained by four disulfide bonds with a complex topology, and its N-terminal end is N-glycosylated at asparagines 331 and 343 (Lan J, et al. Nature. 2020; 581 (7807): 215-20).

Many anti-RBD antibodies block its interaction with ACE2 and therefore neutralize SARS-CoV-2; as a matter of fact, the epitopes of many neutralizing anti-protein S antibodies are located at the RBD (Premkumar L, et al. Sci Immunol. 2020; 5 (48): 1-15). Thus, the RBD itself is considered a promising vaccine candidate not only against SARS-CoV-2, but against other coronaviruses such as SARS and MERS (Zhou Y, Jiang S, Du L. Expert Rev Vaccines 2018; 17(8): 677-86; Wang N, Shang J, Jiang S, Du L. S Front Microbiol. 2020; 11 ). The RBD, for instance, was the first antigen evaluated successfully in phase 1 clinical trials during the SARS-CoV-2 mRNA vaccine development program of BioNTech/Pfizer (Mulligan MJ, et al. medRxiv 2020 Jan 1). According to previous studies with SARS-CoV-1, the risk of immunopathology upon immunization with the RBD is low (Jiang S, He Y, Liu S. Emerg Infect Dis. 2005; 11(7): 1016-20; Jaume M, et al. Hong Kong Med J. 2012; 18 (SUPP2): 31-6). Additionally, the RBD has been successfully expressed in a variety of heterologous systems, ranging from mammalian cells (HEK293, CHO) to methylotrophic yeast such as *Pichia pastoris* (Arbeitman CR, et al. bioRxiv 2020 Jan 1). Lastly, the relatively rigid structure of the RBD, stabilized by four disulfide bonds, confers a high degree of thermotolerance to this antigen, which is an advantage during field use of RBD-based vaccines whenever cold chain failures are anticipated (Malladi SK, et al. bioRxiv 2020 Jan 1).

Although the most studied variant of the SARS-CoV-2 RBD goes from arginine 319 to phenylalanine 541, this variant leaves, in its N-terminus, a strand that forms part of a beta sheet in the native structure, and a free cysteine residue in its C-terminus that may potentially form dimers with other RBD monomers or catalyze disulfide exchanges that may scramble the structure of this molecule, triggering the formation of intermolecular aggregates (Rabdano SO, et al. Sci Rep 2017; 7(1): 1-20). Extended variants such as 319-541 and closely related variations (residues 318-510, residues 319-591, for instance) have the additional disadvantage of potentially lower thermotolerance and higher sensitivity to proteolytic degradation than shorter RBD variants (such as 331-528 and 331-532) due to the additional flexibility these extensions confer to the termini of the protein (Karshikoff A, Nilsson L, Ladenstein R. FEBS J. 2015; 282 (20): 3899-917).

The RBD of protein S plays a fundamental role in the viral replication cycle, as it mediates the interaction of the virus with its cell receptor, ACE2. It is also the target of potent neutralizing antibodies, and has thus been considered an attractive starting point for the development of subunit vaccine candidates, several of which have reached clinical trials.

The RBD is a domain containing approximately 200 amino acids, whose structure is characterized by: a) the presence of a central antiparallel five-strand beta sheet, packed on both sides against segments of small helixes and loops, b) a protuberant beta hairpin loop involved in the interaction with the receptor, c) a small peripheral three-strand mixed beta sheet, d) two N-glycosylation sites, and e) four disulfide bridges.

This domain locates to the topmost position - the protruding upper part- of protein S, where it exhibits a dynamic "breathing" pattern alternating between different protein S conformations. In a conformation known as "all down", the three RBD domains of a protein S trimer exhibit a C3 (pseudo)symmetry, establishing protein-protein and protein-glycan contacts with adjacent RBDs of the two other protein S monomers. In "all down", solvent accessibility of the surface residues of the RBD is minimized, and what does remains accessible is kept largely unable to interact with other proteins owing to the steric hindrance produced by its own glycans. In this conformation the S trimer is unable to interact with the ACE2 receptor because the interacting surface is not accessible, and the trimer is therefore inactive. However, either one, two or all three of the RBD of a spike trimer may adopt the "up" conformation; the resulting trimer conformations are denominated "one up", "two up" and "three up", respectively. The required conformational change involves pivoting around a hinge formed by a flexible region around lysine 529, changing as a consequence the orientation of the RBD with respect to the remainder of protein S, separating it from the protein domain underneath (known as subdomain 1, or SD1) and eliminating the contacts with subunit S2 of the RBD and SD1.

An RBD in "up" conformation exposes to the solvent the receptor interacting surface. Therefore, the biologically active forms of the S trimer -regarding the initial stage of the cell entry process- are those in which at least one RBD is "up". Electron cryomicroscopy has shown that protein S trimers exist in different proportions of mutually interconverting "all down", "one up", "two up" and "all up" conformations. This "breathing" process lets the virus fine-tune the number of active receptor-binding sites per S trimer, balancing the need to bind to ACE2 with the need to escape neutralizing antibodies by hiding the RBD in "down" conformation. Such a strategy is made possible, in the first place, by the strength of the RBD-ACE2 interaction, which exhibits affinity constants around 10 nM, and by the fact that ACE2 exists as a dimer in which each RBD-interacting site is separated by approximately 10 nm, which is approximately the distance separating adjacent S trimers in a SARS-CoV-2 virion. Hence, full occupancy of ACE2 during SARS-CoV-2 binding only requires one RBD in "up" conformation in two adjacent S trimers, yielding a bivalent interaction of very high apparent affinity.

Several corollaries follow from the relationship between structure and function in protein S and its RBD that bear relevance for the design of subunit vaccine candidates based on recombinant RBD. These candidates must be able to elicit a robust antibody response, that is, they must be highly immunogenic, but the quality of the response is also of paramount importance, as it must consist of high-affinity antibodies able to compete with the RBD-ACE2 interaction even in the bivalent interaction scenario outlined above.

The trimeric conformation of protein S, which consists of subunits S1 and S2, is actually a metastable prefusogenic state: binding to ACE2 destabilizes this structure, triggering the loss of subunit S1 -which requires cleavage at the S1/S2 and S2* sites by the furin and TMPRSS2 proteases- and leading to the adoption of a fusogenic structure that exposes the fusion peptide and initiates the membrane fusion process. Consequently, inhibiting the RBD-ACE2 interaction blocks every downstream event of the viral replication cycle and represents the mechanistic basis on which every RBD-based coronavirus vaccine rests.

There are abundant data in the literature supporting the notion that intranasal immunization schemes might be particularly relevant for the prevention of SARS-CoV-2 infections. For instance, it has been shown for both symptomatic and asymptomatic COVID-19 patients that viral loads in nasal swabs are always higher than viral loads in throat swabs, evidencing the central role of the nasal epithelium for the initial events of viral infection and transmission (Zhou P, et al. Nature. 2020 Mar; 579 (7798): 270-3). Also, the genes associated with viral entry in the case of SARS-CoV-2 are detected in specific respiratory, corneal and intestinal cells. In nasal epithelial cells, these genes are co-expressed with genes involved in innate immunity, highlighting the potential role played by these cells in the initial viral infection, propagation and elimination. These data also point to nasal epithelial cells as possible viral reservoirs participating in intra- and inter-individual dissemination (Sungnak W, et al. Nature Medicine. 2020 May; 26(5): 681-7).

According to existing studies, ACE2 may be a limiting factor for SARS-CoV-2 entry, during the initial stages of the viral replication cycle. ACE2 is expressed, although at rather low levels, throughout a diverse range of epithelial cell types all over the respiratory airways, including type II alveolar epithelial cells. Among all these cell types, however, nasal epithelial cells stand out as those exhibiting the highest levels of ACE2 (Sungnak W, et al. Nature Medicine. 2020 May; 26(5): 681-7). The relatively high expression of ACE2 in nasal samples and, in parallel, the high infectivity of cell cultures derived from the nasal epithelium, suggest that the nasal cavity is a fertile site for the early infection by SARS-CoV-2. It is probable that the nasal infection is dominated by ciliated cells in the superficial epithelium. The efficacy of the infectivity / replication of the virus varies notably from the proximal airway to the alveolar respiratory regions (Hou YJ, et al. Cell. 2020 May 27). In summary, since nasal transport is likely to be a key characteristic of SARS-CoV-2 transmission, nasally administrated drugs and vaccines might be very efficient to limit propagation.

On the other hand, the virus-like particle (VLP) of the core antigen of the Hepatitis B Virus (HBcAg) (full-length 183 residue antigen) produced in *Escherichia coli* is an approximately 30 nm nanoparticle (Aguilar JC, Lobaina Y, Muzio V, et al. Immunol Cell Biol 2004; 82: 539-546). Previously, this particle has been shown to exhibit adjuvant or immunoenhancing effects (Riedl P, et al. J Immunol 2002, 168(10): 4951-4959; Vanlandschoot P, Cao T, Leroux-Roels G. Antiviral Res 2003, 60(2): 67-74). The immunoenhancing properties of this nanoparticle are evident when it is co-administered with other antigens, either intranasally (Aguilar JC, et al. Immunol Cell Biol 2004, 82(5): 539-546; Lobaina Y, et al. Mol Immunol 2005, 42(3): 289-294) or parenterally (Riedl P, et al. J Immunol 2002, 168(10): 4951-4959). Besides, it is known that this VLP is very immunogenic in mice, where it induces an immune response similar to that observed in humans (Milich DR, Semin Liver Dis 1991, 11(2): 93-112). Therefore, the results obtained in mice have a considerable predictive value regarding possible results in humans.

One of the strategies currently followed for the prevention of coronavirus infections is the development of subunit vaccines, consisting of the use, as immunogens, of viral proteins obtained by recombinant DNA technology. Among subunit vaccines, several candidates based on the RBD are currently under development or have reached the clinical stage. Although most of these use monomeric RBD, which does not recapitulate the quaternary structure of the Spike protein on the virus and is poorly immunogenic, some candidates employ multimeric constructs, using e.g. fusion proteins where the RBD is linked to trimer-forming coiled-coil segments. However, these constructs also fail to mimic faithfully the spatial relationship between RBD domains in the native Spike trimer or the inherent supramolecular structure of a viral particle. Other strategies are based on chemical conjugation to carrier proteins and/or polymer-based nanoparticles with immunostimulating properties. Carrier proteins, with a wide repertoire of helper T cell epitopes, increase the immunogenicity of their cargo proteins, and the conjugation of carrier to cargo proteins is one of the vaccine design strategies in the state of the art. It must be noted, though, that chemical conjugation leads to the formation of covalent bonds between functional groups on the RBD moiety and the carrier protein or polymer via chemical reactions of an essentially random nature, which may therefore affect important epitopes on the surface of the RBD or the carrier and typically produce highly heterogeneous preparations, requiring further purification steps to remove unwanted reactants and side products.

Due to these reasons and the potential impact of coronavirus infections, the design and production of new vaccine candidates that can efficiently prevent disease produced by this type of viruses continues to be of the utmost importance.

### Detailed description of the invention

The present invention provides solutions to the problems mentioned above through the design of a novel chimeric protein with a modular structure comprising: a) an RBD from a coronavirus protein S that contains a segment of said domain from protein S; b) an HBcAg-binding segment that comprises the amino acid sequence WSFFSNI; c) a segment that comprises the amino acid sequence HHHHHH; d) a flexible spacer segment of 5 to 12 amino acids composed of the amino acids Gly and Ser and e) a 7 to 20 amino acids spacer segment selected between a segment comprising SSSSSSSSSS and the segment STNLAAA. In the chimeric protein disclosed in the invention, segments a) to e) are arranged in order (b)-(d)-(a)-(e)-(c) or (c)-(e)-(a)-(d)-(b). In one embodiment of the invention, the RBD segment of the chimeric protein is the segment comprised between amino acids Q320 to C590 of protein S from SARS-CoV-2. In one embodiment, the flexible 5-12 amino acids spacer segment (d) has a sequence selected from the group composed of sequences GGSGG, GGSSGGS, GGGSSGGG, GGGSSGGSSGGG and GGSGGSGGS. In one embodiment of the invention, the 7-20 amino acids spacer segment (e) has a sequence selected from the group composed of sequences GGSGGSSSSSSSSSSIE, SSGSSSSSSSSSS and GGSGGSSSSSSSSSSGGGIE. In a particular embodiment, the chimeric protein has the amino acid sequence identified as SEQ ID NO: 4 or SEQ ID NO: 5.

The invention provides a vaccine composition comprising: I) the chimeric protein with a modular structure comprising: a) an RBD from a coronavirus protein S that comprises a segment of said domain from protein S; b) an HBcAg-binding segment that comprises the amino acid sequence WSFFSNI; c) a segment that comprises the amino acid sequence HHHHHH; d) a flexible spacer segment of 5 to 12 amino acids composed of the amino acids Gly and Ser; and e) a 7 to 20 amino acids spacer segment selected between a segment comprised SSSSSSSSSS and the segment STNLAAA, wherein segments a) to e) are arranged in order (b)-(d)-(a)-(e)-(c) or (c)-(e)-(a)-(d)-(b), and II) pharmaceutically acceptable excipients or diluents. In an embodiment of the invention, said vaccine composition also comprises a vaccine adjuvant. In a particular embodiment, the vaccine adjuvant is an aluminum salt.

In one embodiment, the vaccine composition conmprises the aforementioned chimeric protein also contains HBcAg. In a preferred embodiment, the chimeric protein and the HBcAg comprised in the vaccine composition are forming hybrid nanoparticles.

In another embodiment, the vaccine composition disclosed in the invention also comprises a second coronavirus antigen. The vaccine composition of the invention can be administered through, the parenteral route, the mucosal route or a combination thereof, although said routes do not limit the scope of the invention.

Another embodiment of this invention is the use of the aforementioned chimeric protein with a modular structure in the manufacture of a vaccine composition for the prevention of coronavirus infections. In one embodiment of the invention, the infection to be prevented with said vaccine composition is caused by SARS-CoV-2. The vaccine composition for the prevention of coronavirus is administered by the parenteral route, the mucosal route, or a combination thereof in a sequential or concomitant manner during the course of an immunization schedule.

In another embodiment, the invention discloses a method for the prevention of an infection caused by a coronavirus, characterized by the administration to an individual of a pharmaceutically effective amount of a vaccine composition comprising the aforementioned chimeric protein of modular structure and pharmaceutically acceptable excipients or diluents. In one embodiment of the prevention method of the invention, the vaccine composition includes HBcAg. In one embodiment of the invention, in said method for the prevention of coronavirus infections the vaccine composition is administered following the parenteral route, the mucosal route, or a combination thereof.

The examples of invention describe the design of chimeric proteins comprising the RBD from SARS-CoV-2 that are able to form a stable corona -by establishing stable non-covalent interactions- over the surface of self-assembled HBcAg nanoparticles, generating a structural and functional mimic of the SARS-CoV-2 viral surface that can elicit a robust immune response via different inoculation routes, including the parenteral and nasal routes.

The RBD-comprising chimeric proteins disclosed in the present invention overcome the limitations of current vaccine candidates. Unlike the latter, the chimeric proteins described in the present invention can mimic at the same time a larger set of the structural attributes that characterize viral spikes, such as a) the stereochemical features of the RBD trimer, as formed by protein S, b) the ability to fit into a supramolecular structure with a spacing only slightly larger than 10 nm, corresponding to the particular hexahedral structural symmetry of coronaviruses, and c) a structural flexibility conferring a conformational dynamic to the RBD that resembles the "breathing" motion of RBDs in a protein S trimer. The RBD-comprising chimeric proteins disclosed by the present invention are able to adopt a nanometric supramolecular structure through their association by specific non-covalent interactions with the spikes of HBcAg (a 20-30 nm nanoparticle that is highly immunogenic and can be used as a carrier protein), yielding a hybrid self-assembled complex or nanoparticle that can elicit a robust immune response when administered either parenterally or through the mucosal route. The invention comprises, in addition, the ability of these chimeric proteins to stabilize, through the formation of coordination bonds with transition metals, both a quaternary structure characterized by the existence of dimers/trimers and the supramolecular structure of said hybrid nanoparticles. The structural properties of the RBD mimicked by the proteins disclosed in this invention are central determinants of the structure-function relationships exhibited by protein S, particularly during binding to its receptor and the formation of a bivalent association with ACE2 dimers. Also, the proteins disclosed in this invention have the capacity to elicit neutralizing antibodies that can bind, in a bivalent fashion, adjacent spikes in the surface of coronaviruses, mimicking the binding interaction with ACE2 dimers and thus exhibiting higher avidity and neutralization potency.

Another novelty of the present invention that relates to the presence of HBcAg in the inner core of the disclosed hybrid nanoparticles is the fact that this antigen, in addition to being highly immunogenic and functional as a carrier protein, is able to induce by itself an innate immune response. Thus, the hybrid nanoparticles disclosed in the present invention not only can generate neutralizing antibodies as part of an RBD-specific response, but can stimulate the innate immune system, which has been shown to be deteriorated in the older age groups that exhibit the highest risk regarding the severity and mortality of COVID-19.

As described in Example 1, the chimeric proteins containing the RBD disclosed in the present invention have a modular structure comprised of five domains or modules with well-defined structural features and functions. The module comprising the coronavirus RBD (a) is the element responsible for generating a specific immune response consisting of neutralizing antibodies against SARS-CoV-2. The remaining modules confer the RBD module the ability to bind the spikes of HBcAg nanoparticles in such a way that a stable corona is formed, mimicking the structural and functional features of the RBD as found in the surface of coronaviruses.

The rational design followed in the present invention enables the precise localization of the chimeric polypeptide to the apex of HBcAg spikes, which constitute the most protruding and immunodominant region of this protein. Other technical solutions for the utilization of HBcAg for similar purposes have been tried previously, but they are inferior to the present invention. For instance, one often used alternative has been the synthesis of fusion proteins wherein heterologous peptides or proteins are inserted into the connecting loop joining the helixes of the HBcAg spike. This alternative is not adequate for proteins whose native conformation requires the formation of disulfide bonds, post-translational modifications such as glycosylation, or simply require transit through the secretory pathway, because HBcAg is a cytoplasmic protein that self-assembles in the cytoplasm, and thus these fusion proteins are unable to fold properly after exiting the ribosome.

The technical solution disclosed in the present invention uses a much simpler design to precisely position the heterologous protein on top of the HBcAg spikes: a non-covalent specific interaction mediated by module (b). Hence, with the present invention the protein cargo can be expressed independently in the most appropriate heterologous host. One example is represented by this invention, where the designed proteins have been synthesized in HEK293 cells, CHO cells, and *Pichia pastoris* strains.

The present invention also overcomes limitations of traditional conjugation methods that rely on covalent bonding. Achieving site-specific chemical conjugation is very difficult, and often the final conjugate is the result of a stochastic process where carrier and cargo are linked together in a variety of manners. In addition, the conjugation reactions frequently require non-physiological solvents or temperatures that may be detrimental to the folding of cargo or carrier, or introduce unwanted chemical modifications into amino acid side chains that may compromise the integrity of specific epitopes, thus affecting the quality of the resulting immune response.

It must be stressed that the insertion of module (b) into chimeric proteins is not sufficient to enable the use of said proteins as efficient HBcAg-bound immunogens.

The affinity of the interaction between the motif in module (b) (the WSFFSNI sequence) and HBcAg is 12 µM, which is rather poor and leads to considerable dissociation of the cargo at low protein concentrations, compromising immunogenicity. This problem is likely the reason why there are no known antecedents of the use of this motif for the purposes disclosed in this invention. Hence, as mentioned before, the inclusion of module (b) by itself does not guarantee an effective association of the chimeric protein to HBcAg. As shown in Example 3, for module (b) to fulfill its role it is necessary to also insert module (d) -a highly flexible and soluble spacer designed in the present invention that connects module (b) to the RBD module- and to use defined experimental conditions (*e.g*. temperature) for the association to take place, and neither of these conditions are known in the state of the art.

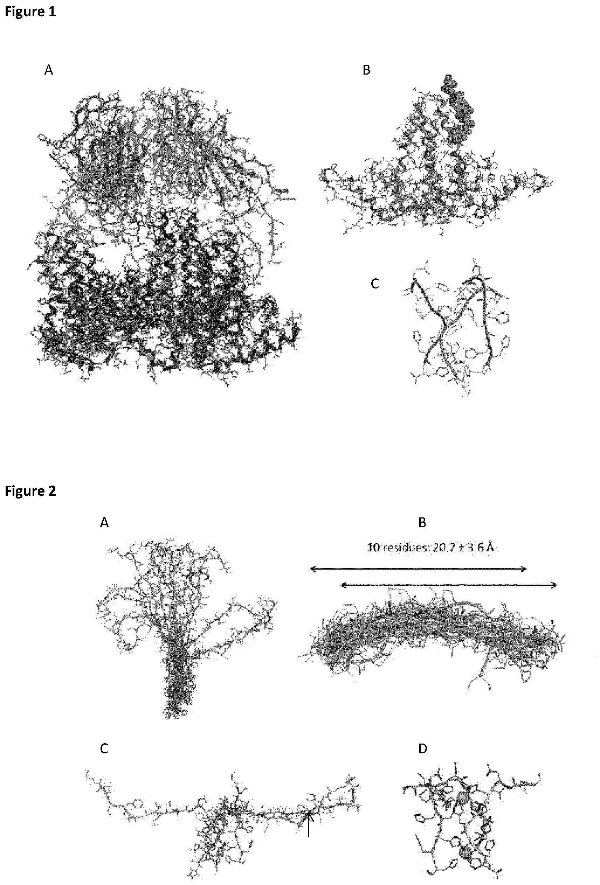

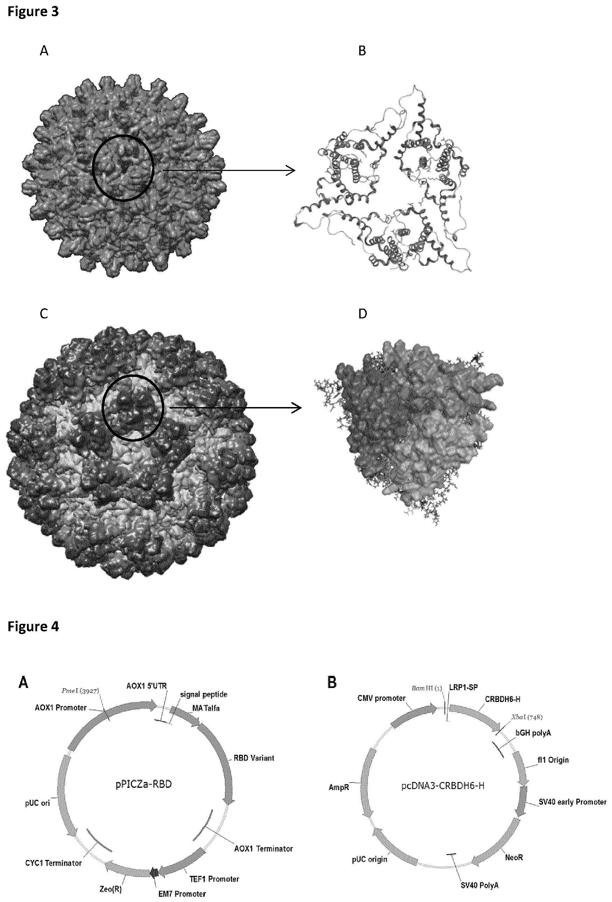

In order to further increase the affinity of the interaction and the stability of the resulting complexes, the present invention also discloses the design of modules (c), (d) and (e), which confer stereo-chemical elements for the intrinsic and extrinsic stabilization of the resulting hybrid nanoparticles (Example 1). The first, central element of the invention rests on the observation that the spatial arrangement of the tips of three adjacent HBcAg spikes coincides, to a large extent, with the spatial arrangement of the N- and C-termini of the RBDs in a protein S trimer, as can be observed on the 3D structure of protein S from coronaviruses (Example 1, Figure 1A and Figure 3). Module (d) was designed to be highly flexible, providing the necessary distance for the RBD module to rise above HBcAg spikes (Figure 1A, Figure 3). The flexibility of module (d) confers to the RBD segment the capacity of structural adaptation via interactions with adjacent RBD segments, in a fashion not dissimilar to that of the "all down" conformation of protein S trimers, and also the ability to change orientation dynamically as in the "up" conformations of protein S trimers. These inter-RBD segment interactions represent the first intrinsic stabilization element disclosed in the present invention and are responsible for the submicromolar stability of hybrid nanoparticles shown in Example 3, which documents an increase in affinity higher than 50-fold over that provided by module (b) alone in a synthetic peptide.

Another experimental demonstration of the stability of the corona designed in the present invention is its capacity to inhibit the binding to HBcAg of polyclonal anti-HBcAg antibodies. This is a highly stringent test due to the high avidity typically exhibited by polyclonal sera, and since the HBcAg spikes constitute the immunodominant epitope of this antigen, it constitutes an additional confirmation of the correct positioning of the chimeric proteins.

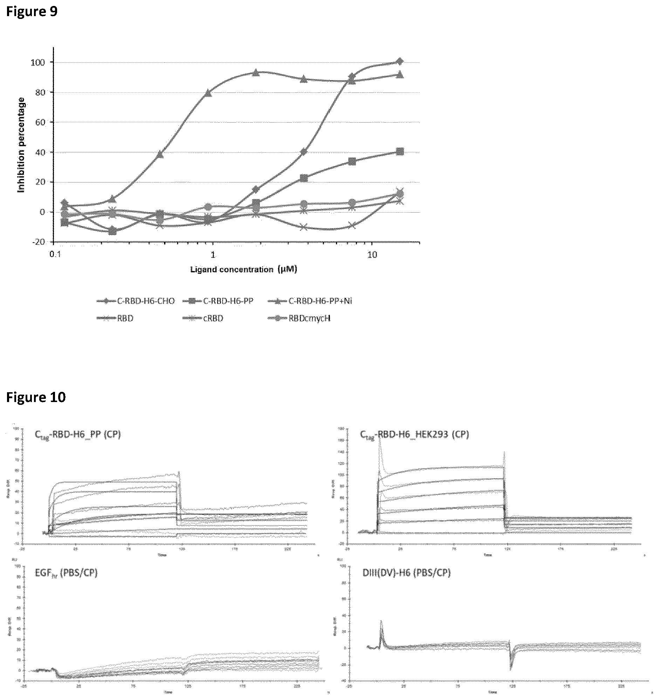

The present invention also employs a strategy of extrinsic stabilization, represented by the presence of modules (c) and (e). Module (c) contains a hexahistidine motif that can form coordination bonds with transition metals and hence form dimers/trimers (Figure 2). Module (e) is a flexible, extended spacer containing a poly-serine segment that connects (c) to the RBD segment and is designed to enable the dimerization/trimerization of the chimeric proteins disclosed in the present invention in the inter-spike space (Figure 2). Example 3 evidences the efficiency of extrinsic stabilization as disclosed in the present invention, depicting how the addition of Ni increases to submicromolar levels the inhibitory effect of the chimeric protein on the binding of anti-HBcAg antibodies (Figure 9).

The designed chimeric protein comprising the RBD binds the ACE2 receptor with high affinity (Example 4), demonstrating that the modular design disclosed by the present invention does not affect in a significant manner the biological function of the RBD segment. This finding is consistent with the fact that non-RBD modules, incorporated as N- and C-terminal extensions to the RBD segment, locate to the portion of the molecule farthest from its receptor-binding surface when the chimeric protein folds. Also, Example 3, where the chimeric protein is incubated at 50°C for 2 hours and then shown to retain its chromatographic characteristics and even increase its HBcAg-binding capacity, proves that the addition of the modules disclosed in the present invention does not have a negative effect on the folding of the chimeric protein.

Example 4 also demonstrates that the hybrid nanoparticles disclosed in the present invention bind efficiently the ACE2 receptor, indicating that said nanoparticles represent a faithful molecular mimic of the viral surface and that the novel modular design of the chimeric protein disclosed in the present invention, which contains the RBD, recapitulates efficiently the essential traits of the structure-function relationship of the RBD from protein S of the SARS-CoV-2 virus.

In the present invention, modules (b) and/or (c) and the corresponding spacers connecting them (d) and (c) are also employed as elements stabilizing the intrinsic interactions of chimeric RBD proteins in solution, even in the absence of HBcAg particles. Since the amino acid composition of module (b), containing three aromatic residues, three polar residues and one hydrophobic residue is typical of protein-protein and protein-glycan interacting surfaces, said module may establish additional intra- and inter-molecular interactions that stabilize the formation of dimers, trimers and oligomers of RBD-containing chimeric proteins. These interactions are potentially facilitated by the flexibility of these modular spacers and, in the case of module (e), the presence of a poly-serine stretch that is not only able to form hydrogen bonds but is commonly associated with protein-glycan interactions.

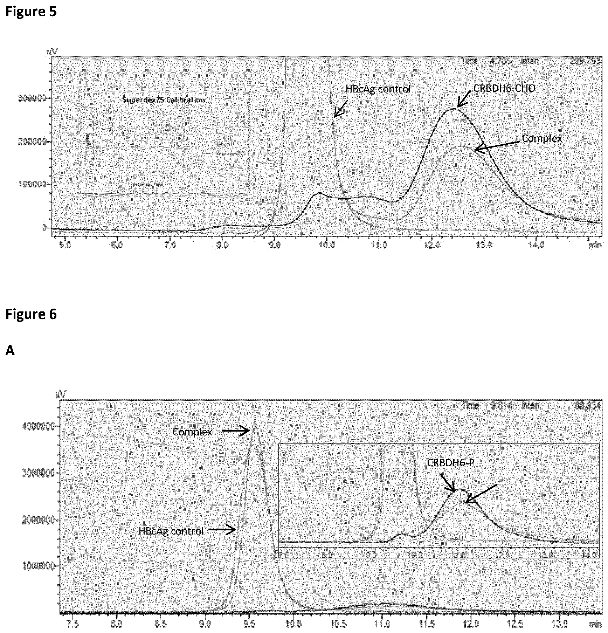

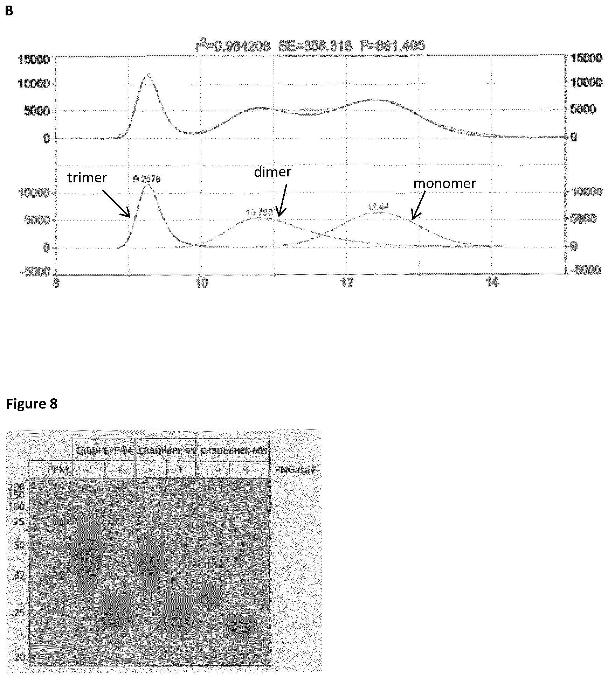

Example 3 depicts evidence of the existence of the aforementioned additional stabilizing interactions: i) protein CRBDH6-P exists mainly as a monomer in solution at submicromolar concentrations, but a minor fraction corresponding to protein trimers or higher-order aggregates is also detectable (Figure 6A); ii) likewise, although the monomeric fraction is predominant in protein CRBDH6-CHO (chimeric protein CRBDH6-H expressed in CHO cells), dimers and trimers can also be observed (Figure 5); and iii) the sum of the dimeric and trimeric fractions, in the case of protein CRBDH6-H, is larger than the monomeric fraction (Figure 7B). In turn, the relative abundance of the different species can be modulated through extrinsic interactions mediated by the presence of the hexahistidine in module (c), which can form coordination bonds with transition metals and thus further stabilize the formation of dimers and/or trimers. Protein CRBDH6-CHO is the same polypeptide as protein CRBDH6-H, but produced in the CHO cell line.

The above highlights, as an element of novelty in the present invention, the ability of the designed molecules to form diverse quaternary structures, which is a desirable characteristic for vaccine preparations based on the RBD of protein S. The presence of diverse quaternary structures means that the immune system is faced with a structural variety of said domains, consistent with the different "up" and "down" states of the RBD in its native context within the coronavirus protein S spike. Example 5 demonstrates how immunization with the RBD segment alone, without the other modules disclosed in the present invention, elicits an immune response that is clearly inferior to that elicited by the chimeric proteins with all five segments as disclosed in the present invention.

On the other hand, in the present invention, as part of Example 5, two variants of the protein S fragment from SARS-CoV-2 described as RBD and expressed in the yeast *Pichia pastoris* were also evaluated. One of the evaluated variants contained a hexahistidine tag on its C-terminal end, and it was possible to induce the dimerization/trimerization of said variant by using metal ions. The obtained results indicate that the neutralization titers of the antibody response obtained by immunization with said yeast-expressed dimerized/trimerized RBD are higher than those obtained by immunization with the yeast-expressed monomeric RBD variant. This result is consistent with the dimer/trimer mimicking more accurately the quaternary structure of protein S as exposed in the virion surface, which mediates binding to the ACE2 receptor (Wrapp et al., Science 367, 1260-1263 (2020)).

The results obtained in Examples 6 and 7 of the present invention represent a novel solution to the prevailing problem in the state of the art about the need for new formulations enabling the potentiation of the immune response against antigens of interest for vaccines in order to obtain immunogens that are effective for the control of diseases and infections. Taking as an example recombinant RBD variants expressed in two different hosts (i.e., *P. pastoris* and the HEK-293 cell line), in both cases the immunogenicity of preparations containing hybrid HBcAg/RBD nanoparticles was higher, regardless of immunization route (intramuscular or intranasal). Also, the functionality of the antibodies developed in the sera of the animals was verified in an assay for the inhibition of the interaction of RBD with the ACE2 viral receptor, which was corroborated with a neutralization assay in the case of animals immunized intramuscularly. The results indicate that the high density of exposed RBD in the complexes formed over the surface of the HBcAg nanoparticle contributed to raise the immunogenicity above the other evaluated groups. The co-administration of these antigens without association does not explain it, since the groups immunized intramuscularly with both antigens without association, although they elevated their humoral response with respect to those where HBcAg was not co-administered, generated a level of titers that was significantly inferior. That is, an adjuvant effect of the HBcAg nanoparticle does not account for the result obtained with hybrid HBcAg-CRBDH6 nanoparticles. In this sense, the design of the hybrid HBcAg-CRBDH6 leads to the orderly anchoring of chimeric CRBDH6 protein molecules to the spikes of the HBcAg nanoparticles, whose spatial distribution has been shown to play a role in the T-independent stimulation of anti-HBcAg B-lymphocyte clones (Milich DR, McLachlan A. Science 1986; 234: 1398-1401). On the other hand, the fact that only the hybrid HBcAg-CRBDH6 nanoparticle elicited an IgA response of significant levels at the nasopharyngeal mucosa of 100% of the immunized animals evidences that such hybrid nanoparticles -nanometer-sized- and all the other aforementioned characteristics, were highly efficient for stimulating the cells of the mucosal immune system. These are novel results.

### Brief description of the figures

**Figure 1****.** Schematic representation of the 3D structure of a chimeric RBD protein forming a complex with HBcAg spikes and establishing an intrinsic and extrinsic stabilization network. **A)** The upper half depicts a trimer of protein CRBDH6-P adopting a conformation akin to the "all-down" conformation of protein S trimers in SARS-CoV-2 virions. Each chain is colored with different tones of gray, and the dark grey helixes of the bottom part correspond to three adjacent spikes on the surface of the HBcAg nanoparticle. Each HBcAg spike is non-covalently bonded to a single chain of chimeric RBD protein. In the central space between the spikes and underneath the RBDs (modules (a)) the figure shows three hexahistidine segments (modules (c)) coordinating two Ni atoms, represented as two spheres. **B)** 3D model of the interaction between a module (b), containing the HBcAg-binding motif, with a spike of the HBcAg nanoparticle. Module (b) is represented with spheres, and the helixes correspond to the dimeric HBcAg spike. **C)** 3D model of a hexahistidine trimer (module (c)) coordinating to Ni atoms (spheres).
**Figure 2****.** Structural analysis of the design of modules (c) and (e). **A)** Superposition of twenty 3D models of segment Ser₁₇₀-His₁₈₈ in the N-terminal portion of protein CG2496 (PDB identifier 2kpt), whose structure in solution was solved by NMR. This segment has a sequence similar to modules (e)-(c) disclosed in the present invention: SSSSSSSGSSSLEHHHHHH. Only the coordinates for the hexahistidine segment were considered for the superposition. As the figure shows, the poly-serine segment takes a variety of orientations relative to the hexahistidine segment. **B)** Superposition of twenty models of the Ser₁₇₀-Ser₁₈₀ segment. The mean distance between the N-and C-termini of the 3D models of the Ser₁₇₀-Ser₁₇₉ and Ser₁₇₁-Ser₁₈₀ decapeptides is 20.7 Å. The poly-serine segment is flexible but adopts an extended conformation, with a well-defined unidirectional advance. **C)** Trimer formed by the Tyr₄₈₁-His₅₉₂ segment of the N-terminal domain of the Na-K ATPase (PDB entry 1q3i). This trimer forms in a C3 crystallographic symmetry center and its formation is facilitated by the coordination of two Ni atoms (spheres) by the C-terminal hexahistidine segment of the protein. The figure also shows (identified with an arrow), superposed onto the above trimer and identified with an arrow, one of the 3D models of the Ser₁₇₀-His₁₈₈ segment from Figure 2A which, despite not being associated with a Ni atom, exhibits a conformation similar to those of the coordinated hexahistidine moieties. **D)** Structure of the coordination complex formed by two Ni atoms and three hexahistidine segments from Figure 2C.
**Figure 3****.** Diagram of the corona surrounding the HBcAg nanoparticle, formed by the non-covalent association of protein CRBDH6-P. **A)** Naked HBcAg nanoparticle. The circle surrounds three adjacent HBcAg spikes, each formed by an HBcAg dimer. **B)** Enlarged representation of the three spikes within the circle in Figure 3A where the helical structure of the six chains (three HBcAg dimers) is easily observed. **C)** 3D model of a hybrid nanoparticle. The corona formed by protein CRBDH6-P trimers is colored in dark gray (the circle surrounds one of them) and the surface of the HBcAg nanoparticle underneath the corona layer is colored in light grey. **D)** Model of a CRBDH6-P trimer forming a complex above three HBcAg spikes. In this particular case the RBDs are adopting an "all-down" conformation similar to the homonymous conformation of protein S trimers in SARS-CoV-2 virions.
**Figure 4****.** Plasmids resulting from the ligation of fragments coding for the different RBD variants into pPICZalfaA (**panel A,** variants: RBD, RBDH, RBDcmycH, CRBDH6-P and CRBD) or into pcDNA3 (**panel B,** CRBDH6-H variant).
**Figure 5****.** Examination by gel filtration chromatography of the association of protein CRBDH6-CHO to HBcAg nanoparticles. The inset depicts data from the calibration of this column with the low molecular weight calibration kit from General Electric^{™}. The figure shows the chromatographic profiles of: the HBcAg control, protein CRBDH6-CHO, and their complex. Run time is indicated on the horizontal axis and absorbance on the vertical axis. Every sample was pre-incubated at 37°C for 2 hours.

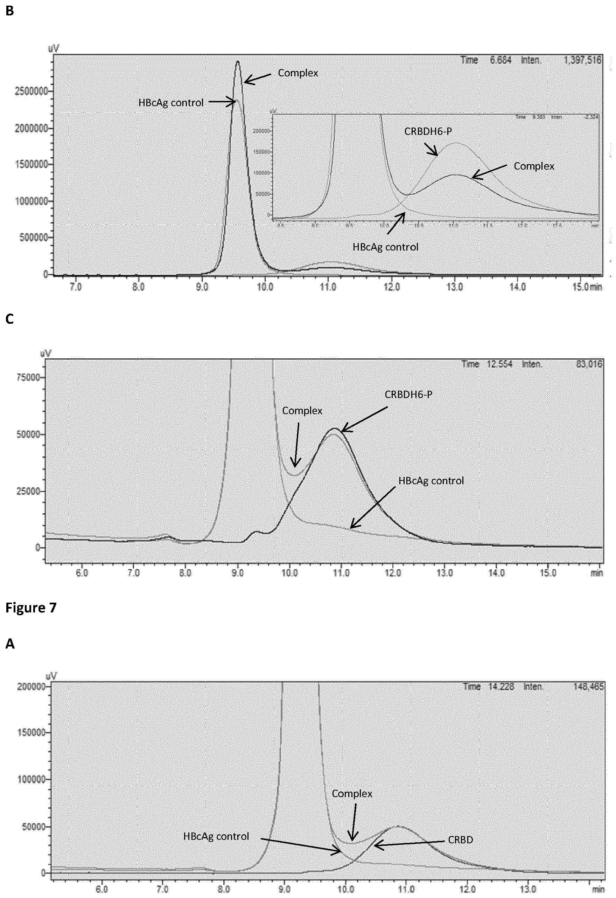

**Figure 6****.** Study by gel filtration chromatography of the association of protein CRBDH6-P to HBcAg nanoparticles. The figure shows the chromatographic profiles of the HBcAg control, protein CRBDH6-P, and the HBcAg/CRBDH6-P complex. The horizontal axis corresponds to the run time and the vertical axis to absorbance. **A)** Samples pre-incubated at 37°C for 2 hours. The inset presents the CRBDH6-P portion of the chromatogram with different scaling for easier observation of the CRBDH6-P peak. **B)** Samples pre-incubated at 50°C for 2 hours. The inset presents the CRBDH6-P portion of the chromatogram with different scaling for easier observation of the CRBDH6-P peak. **C)** Samples pre-incubated at 18°C for 2 hours.
**Figure 7. A)** Study by gel filtration chromatography of the association of protein CRBD to HBcAg nanoparticles. The figure shows the chromatographic profiles of the HBcAg control, protein CRBD, and their complex. The horizontal axis corresponds to the run time and the vertical axis to absorbance. All the samples were pre-incubated at 37°C for 2 hours. **B)** Study by gel filtration chromatography of the quaternary structure of protein CRBDH6-H. The top half of the chart shows the chromatogram and the bottom part its deconvolution, yielding three peaks that correspond to monomeric, dimeric and trimeric species.
**Figure 8****.** Analysis by 12.5% SDS-PAGE, under reducing conditions, of two preparations of the recombinant CRBDH6-P chimeric protein (produced in *Pichia pastoris*) and two preparations of the recombinant CRBDH6-H chimeric protein (produced in the HEK293 cell line). PPM: Molecular weight standards. The PNGase F (-) lanes contain the native preparations and the PNGase F (+) lanes contain preparations that have been N-deglycosylated with PNGase F.
**Figure 9****.** Assay for the inhibition of the binding of polyclonal anti-HBcAg antibodies to hybrid nanoparticles formed by the chimeric RBD proteins of the present invention and HBcAg.
**Figure 10****.** Sensorgrams of the determinations for chimeric proteins CRBDH6-P and CRBDH6-H performed on a BIACORE X sensor, employing as immobilized ligand, on the surface of a CM5 chip, the chimeric protein ACE2-mFc, obtained by recombinant DNA techniques in a mammalian cell line (HEK293). **Top row:** CRBDH6-P and CRBDH6-H dissolved in citrate (17 mM) / phosphate (66 mM) buffer at pH 6.2; **Bottom row:** Proteins used as negative controls of the experiment: Epidermal Growth Factor (EGFhr, left) and recombinant domain III from the Envelope protein of Dengue virus (DIII(DV)-H6, right).

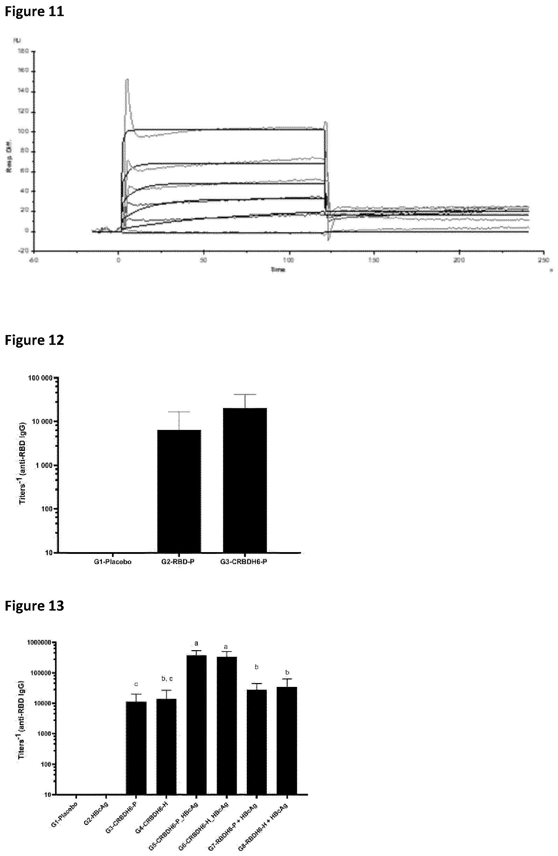

**Figure 11****.** Sensorgrams of the binding of hybrid HBcAg-CRBDH6-H nanoparticles to the ACE2-Fc receptor in citrate/phosphate buffer (CP pH 6.2). The estimated equilibrium constant is KD = 2.94 × 10⁻¹¹ M (Chi² = 9.75). The ligand immobilized on the CM5 chip and the immobilization conditions are identical to those of Figure 10.
**Figure 12****.** Humoral anti-RBD IgG response in serum. Balb/c mice were immunized at days 0 and 14 via i.m. with the immunogens described on the horizontal axis. Serum samples were collected 10 days after the second dose, and their titers were determined by ELISA. The chart depicts geometric means and their 95% confidence interval.
**Figure 13****.** Humoral anti-RBD IgG response in serum. Balb/c mice were immunized at days 0 and 14 via i.m. with the immunogens described on the horizontal axis. Serum samples were collected 10 days after the second dose, and their titers were determined by ELISA. Different letters indicate statistically significant differences (p<0.05). The chart depicts geometric means and their 95% confidence interval.

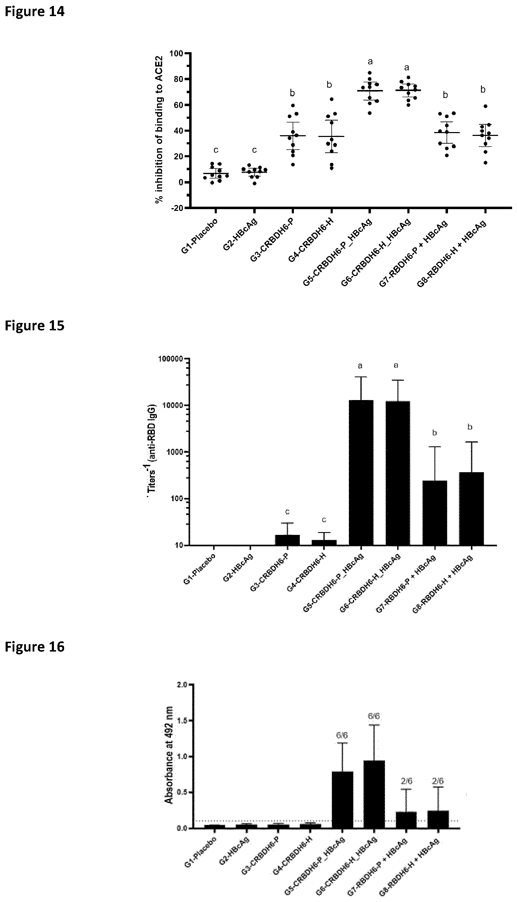

**Figure 14****.** Assay for inhibition of the RBD-ACE2 interaction by anti-RBD sera. Balb/c mice were immunized at days 0 and 14 via i.m. with the immunogens described in the horizontal axis. Serum samples were collected 10 days after the second dose and diluted 1:200 for the assay. Different letters indicate statistically significant differences (p<0.05). The chart depicts means and their 95% confidence interval.
**Figure 15****.** Humoral anti-RBD IgG response in serum. Balb/c mice were immunized at days 0 and 14 via i.n. with the immunogens described in the horizontal axis. Serum samples were collected 10 days after the second dose and the titers were determined by ELISA. Different letters indicate statistically significant differences (*p*<0.05). The chart depicts geometric means and their 95% confidence interval.
**Figure 16****.** Humoral anti-RBD IgA response in nasopharyngeal washes. Balb/c mice were immunized at days 0 and 14 via i.n. with the immunogens described in the horizontal axis. The intranasal washes were collected 10 days after the second dose and their absorbance was determined by ELISA. The frequency of responder animals is indicated on top of each column. The dashed line indicates the positivity threshold. The chart depicts means and their 95% confidence interval.

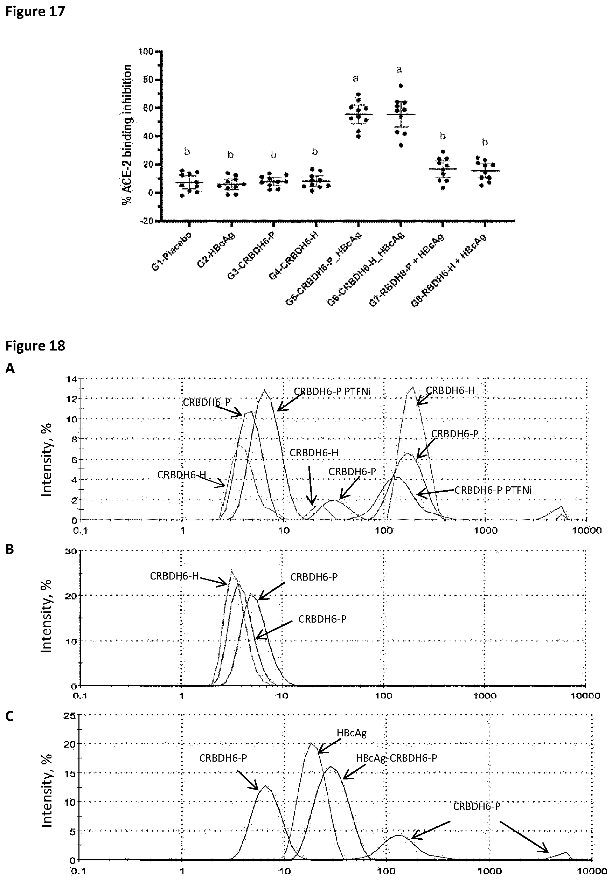

**Figure 17****.** Assay for inhibition of the RBD-ACE2 interaction by immune sera. Balb/c mice were immunized intranasally at days 0 and 14 with the immunogens described in the horizontal axis. Serum samples were collected 10 days after the second dose and diluted 1:200 for the assay. Different letters indicate statistically significant differences (*p*<0.05). The chart depicts means and their 95% confidence interval.

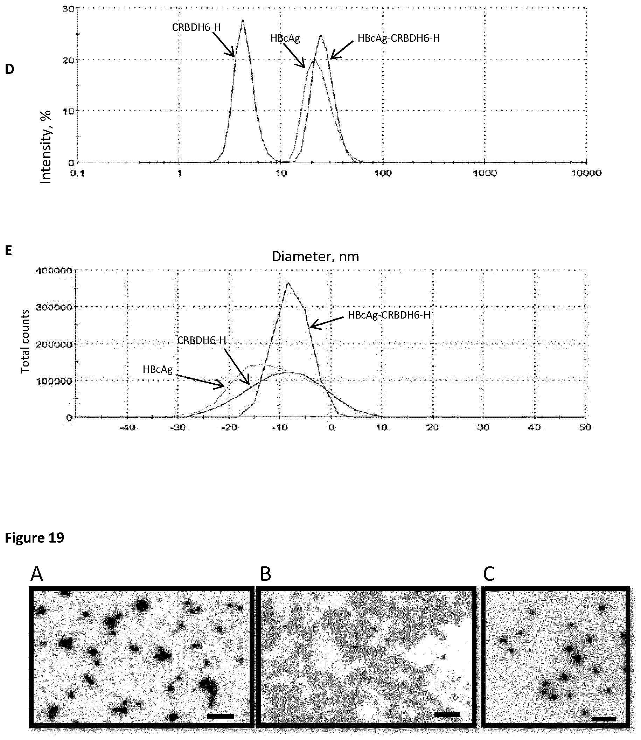

**Figure 18****.** Analysis by Dynamic Light Scattering (DLS) spectroscopy of the quaternary and supramolecular structure of proteins CRBDH6-H (**A, B** and **D**) and CRBDH6-P (**A, B** and **C**), as well as of the formation of the hybrid HBcAg-CRBDH6-P (**C**) and HBcAg-CRBDH6-H (**D**) nanoparticles. Panels **A** and **C** show the size distribution by intensity, and panels **B and D** show the size distribution by volume. In panels **A, B** and **C,** CRBDH6-H and CRBDH6-P refer to these proteins dissolved in PBS 1X pH 7.4 and PBS 0.3X pH 7.4, respectively. CRBDH6-P-PTFNi refers to CRBDH6-P dissolved in PBS/Tween-20 0.03%, fructose 12%, NiSO₄ 1 mM pH 7.4; HBcAg refers to HBcAg dissolved in PBS/Tween 0.03% pH 7.4, and HBcAg-CRBDH6-P is the DLS spectrum obtained by mixing HBcAg and CRBDH6-P-PTFNi. In panel **D,** all the samples are dissolved in 20 mM Tris pH 7.4, fructose 12%, NiSO₄ 1 mM. Panel **E** shows the distribution of the zeta potential of the samples from panel **D.**

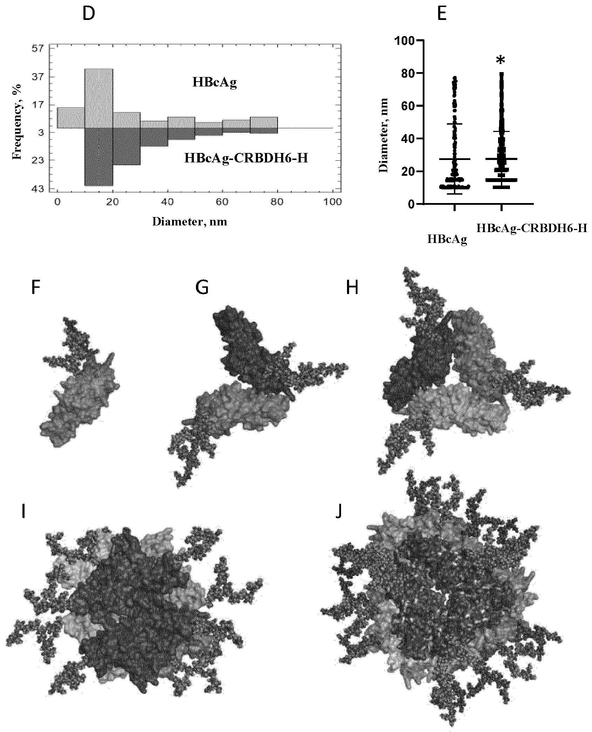

**Figure 19****.** Analysis by Transmission Electron Microscopy (**A-E**) of the hybrid HBcAg-CRBDH6-H nanoparticle, the HBcAg nanoparticle, and protein CRBDH6-H. **A-C:** Microphotographies of HBcAg-CRBDH6-H, CRBDH6-H and HBcAg, respectively. **D:** Comparison of particle diameter histograms of the hybrid HBcAg-CRBDH6-H nanoparticle and the HBcAg nanoparticle. **E:** Chart depicting the observed particle diameter values in both cases. **F-J:** Representation of the 3D structure of glycosylated CRBDH6 proteins. The figure shows the monomer (**F**), dimer (**G**), trimer (**H**), hexamer (**I**) and dodecamer (**J**). The polypeptide chains are depicted in surface representation while the glycans are depicted in a sphere atomic representation.

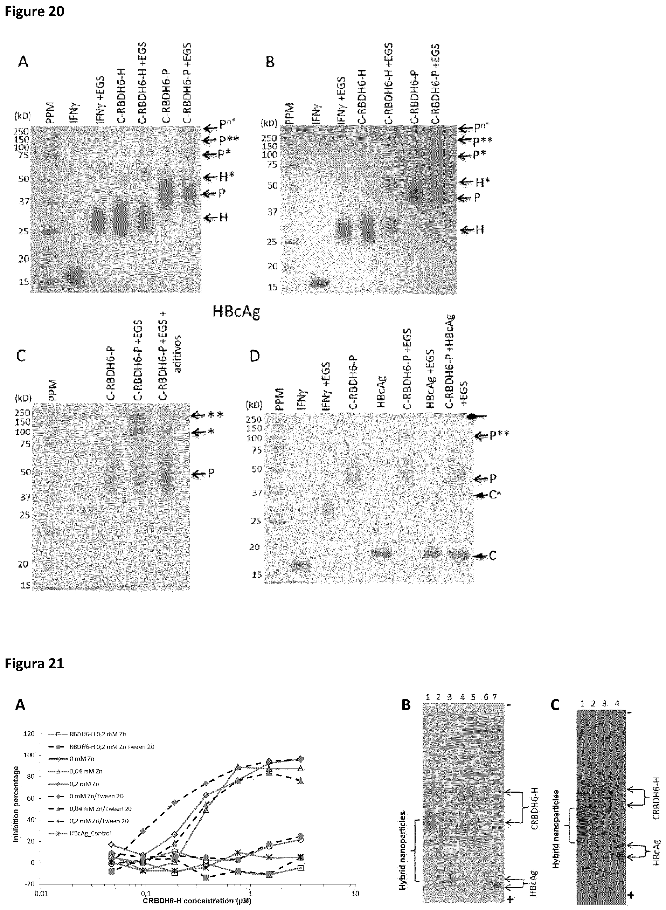

**Figure 20****.** Analysis by SDS-PAGE of the treatment of proteins CRBDH6-H and CRBDH6-P as well as the HBcAg-CRBDH6-P nanoparticle with ethylene glycol bis(sulfosuccinimidyl succinate) (EGS), a cross-linker. The arrows point at the dimeric (*), trimeric (**) and multimeric (n*) species of each protein: H (CRBDH6-H), P (CRBDH6-P) and C (HBcAg). A round-headed arrow points at the location of HBcAg-CRBDH6-P nanoparticle.
**Figure 21****.** Analysis of the formation and stability of hybrid HBcAg-CRBDH6 nanoparticles. **A)** Inhibition curves in competition ELISA showing that hybrid HBcAg-CRBDH6 nanoparticles inhibit the interaction of HBcAg with the anti-HBcAg antibody immobilized on the solid surface of the ELISA plate. **B)** Analysis by native agarose gel electrophoresis of hybrid HBcAg-CRBDH6-H nanoparticles formed in the presence of the Zn⁺² metal ion. Lane 1: Association of the chimeric protein CRBDH6-H to HBcAg using a molar CRBDH6:HBcAg ratio of 2:1; Lane 2: Association of the chimeric protein CRBDH6-H to HBcAg using a molar CRBDH6:HBcAg ratio of 1:1; Lane 3: Association of the chimeric protein CRBDH6-H to HBcAg using a molar CRBDH6:HBcAg ratio of 0.5:1; Lane 4: Control containing only the CRBDH6-H chimeric protein at the same concentration employed for the sample with a CRBDH6:HBcAg molar ratio of 2:1; Lane 5: Control containing only the CRBDH6-H chimeric protein at the same concentration employed for the sample with a CRBDH6:HBcAg molar ratio of 1:1; Lane 6: Control containing only the CRBDH6-H chimeric protein at the same concentration employed for the sample with a CRBDH6:HBcAg molar ratio of 0.5:1, and Lane 7: Control containing only HBcAg at the same, fixed concentration employed for all association reactions. **C)** Analysis by native agarose gel electrophoresis of nanoparticles of the chimeric protein CRBDH6-H formed in the presence of stearic acid. Lane 1: Hybrid HBcAg-CRBDH6 nanoparticle formed in the presence of stearic acid; Lane 2: Nanoparticles of the chimeric protein CRBDH6 formed in the presence of stearic acid; Lane 3: Control containing free CRBDH6-H, and Lane 4: HBcAg control.

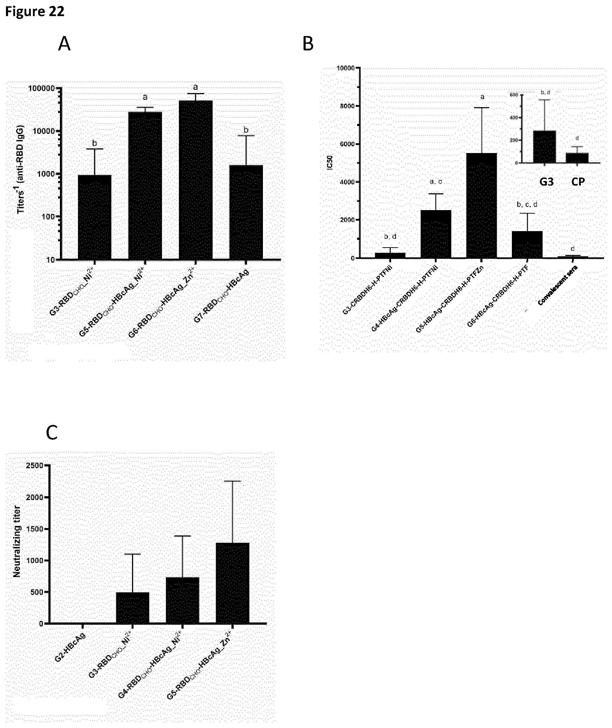

**Figure 22****.** Study of the humoral immune response of mice immunized with hybrid HBcAg-CRBDH6-H nanoparticles. A) Anti-RBD IgG titers (reciprocal of serum dilution); B) Assay for inhibition of the binding of RBD to the ACE2 receptor (IC50 values are expressed as the reciprocal of the serum dilution), the inset compares the inhibitory capacity of anti-CRBDH6-H sera (G3) and of a panel of convalescent sera (CP) *In vitro* virus neutralization assay.

### Examples of realization

### Example 1. Design of chimeric proteins containing the RBD able to mimic the quaternary and supramolecular structure of the spike protein from SARS-CoV-2. Design of hybrid HBcAg-CRBDH6 nanoparticles assembled via highly stable non-covalent interactions

The chimeric RBD proteins of the present invention have a modular structure, and bear:
a) An RBD module containing residues Asn₃₃₁-Lys₅₂₉ from protein S, which consists of segments comprised within the region of protein S delimited by residues Gln₃₂₀ and Cys₅₉₀. The RBD module occupies a central position in the sequence of the chimeric protein, joined to the modules described in (b) and (c) by flexible, polar spacers described in (d) and (e).
b) A module with the capacity of binding specifically and non-covalently to protein HBcAg. In the present invention this module consists of the seven-residue sequence WSFFSNI. This module can be located, relative to the RBD module, in an N-terminal or C-terminal position, and is joined to the RBD module via the spacer module described in (d).
c) A hexahistidine segment. This module can be located, relative to the RBD module, in either an N-terminal or a C-terminal position, and is joined to the RBD module via the spacer module described in (e).
d) A 5-12 residue long flexible spacer segment composed of the amino acids glycine and serine. The amino acid sequence of this segment consists of one of the following sequences: GGSGG, GGSSGGS, GGGSSGGG, GGGSSGGSSGGG, GGSGGSGGS.
e) A spacer segment of 7 to 20 residues, preferably with a propensity to fold in flexible, extended conformations. The amino acid sequence of this spacer module consists of one of the following sequences: GGSGGSSSSSSSSSSIE, SSGSSSSSSSSSS, STNLAAA, GGSGGSSSSSSSSSSGGGIE.

The sequences of the chimeric proteins disclosed in the present invention may be described -according to their modular structure- in one of the following manners: **(b)-(d)-(a)-(e)-(c)** or **(c)-(e)-(a)-(d)-(b)**

### Rationale for the modular structure of the chimeric proteins

Module (a) includes the smaller Asn₃₃₁-Lys₅₂₉ segment from the RBD, said segment constituting an independently folded domain that contains: four disulfide-bonded cysteines, two N-glycosylation sites (Asn₃₃₁ and Asn₃₄₃) and the receptor-binding motif located between residues Asn₄₃₉ and Tyr₅₀₅, which are essential from a functional standpoint. This domain is the target of neutralizing antibodies, whose main neutralization mechanism is the inhibition of receptor binding. The invention also comprises N- and C-terminal extensions to this core domain (Gln₃₂₀-Cys₅₉₀) that include the SD1 structural domain and also contain other epitopes bound by neutralizing antibodies. These extensions, in addition, include the hinge region that is fundamental in the conformational change enabling the RBD to adopt the active "up" conformations of protein S, which is therefore a potential target of neutralizing antibodies.

Module (b) guarantees the association of the RBD module to the HBcAg nanoparticle, which is highly immunogenic and can induce a potent immune response by either parenteral or nasal inoculation. Mere association with HBcAg, however, does not guarantee that the latter will function as a carrier protein that will stimulate a T-helper response, and therefore does not represent in and of itself an straightforward solution. The stoichiometry of the association matters, because if insufficient RBD is present the balance of the resulting immune response can be tipped towards HBcAg and the generation of anti-RBD antibodies will be poor as a consequence. Therefore, an original contribution of the present invention is the combination of module (b) with the (d) spacer modules, which let the RBD modules protrude above the HBcAg spikes and therefore become the immunodominant region of the hybrid nanoparticle. The defining trait of modules (d) is the presence of glycine and serine residues, which have small side chains that confer flexibility (glycine) and solubility (serine) to said modules. Glycines are the predominant residues of modules (d), enhancing their flexibility and minimizing the introduction of additional constraints to the fitting of RBD modules above the HBcAg nanoparticle spikes. The different sizes of the (d) modules disclosed here offer those practicing the present invention the ability to increase the protuberance of the RBD modules and the flexibility of their movement by choosing (d) modules of larger sizes.

In this case we choose to associate the RBD module to HBcAg using specific non-covalent interactions mediated by (b) modules, instead of more commonly used strategies based on chemical conjugation via covalent bonds. The technical solution disclosed in the present invention has the following advantages over more conventional solutions:
1) It does not need chemical reactions, which require the presence of additional downstream purification processes.
2) The possibility that the solvents often required by the abovementioned chemical reactions may affect the structure of the HBcAg nanoparticle and/or the RBD protein is eliminated.
3) Module (b) binds specifically a patch located in the spikes of the HBcAg nanoparticle, producing hybrid nanoparticles with a defined stoichiometry where the RBD protein is positioned above the immunodominant epitope of HBcAg, which is the most protruding region of the nanoparticle (Figure 1 and 3). Chemical conjugation, on the other hand, cannot reach this level of specificity, as it usually proceeds by a stochastic process whereby different residues from both antigens are bonded.
4) The non-covalent association process is performed under physiological conditions and does not require further downstream purification. The amount of associated RBD can be modulated by changing the concentration of the reactants based on their association constant.
5) The covalent conjugation process may introduce chemical modifications into the side chains of the constituting residues of both antigens, which may affect their antigenicity, immunogenicity and recognition of neutralizing epitopes, especially when the number of modified residues is not controlled.

However, the main potential obstacle when using the non-covalent interaction of segment (b) to HBcAg to prepare hybrid nanoparticles is the high probability of dissociation at physiologically relevant concentrations. Such a dissociation process would destroy the ability of HBcAg to act as a carrier protein, thus redirecting the immune response toward the HBcAg antigen due to its high immunogenicity. Particularly, the affinity of the binding of module (b) to HBcAg is low (12 µM), which is probably the fundamental reason why such sequences have never been exploited in practical applications, such as a method of non-covalent "conjugation" for the preparation of immunogens.

The present invention provides an original technical solution to counteract the apparent weakness of the interaction of modules (b) with HBcAg. One central element is the introduction of a network of additional interactions between the different chains of chimeric RBD proteins, conferring a high stability that guarantees that dissociation will remain very low. This network has two components: one, intrinsic, derived from the RBD segments themselves and the appropriate design of segments (d), and another, extrinsic, introduced through the design of the modules (c) and (e) described herein.

The intrinsic component arises from the capacity of RBD segments to establish protein-protein and protein-glycan interactions when at least two adjacent RBD domains coexist in the "down" conformation in a protein S trimer. An essential element of novelty lies in the fact that it is the choice of an HBcAg nanoparticle, together with an appropriate design of modules (b) and (d) that enables the presentation of RBD segments in a quaternary structural context mimicking that of protein S trimers (Figure 1-3). The distance between HBcAg spikes is approximately 4 nm, which is very similar to the observed distance between the corresponding residues of the N- (and C-) termini of adjacent RBDs in the structure of protein S. Owing to this choice, the RBD, once bound to the HBcAg spikes, can follow a spatial arrangement very similar to that observed in SARS-CoV-2 virions.

These characteristics of the hybrid nanoparticles disclosed in the present invention make them superior to other technical solutions previously disclosed during the design of dimeric and trimeric RBD vaccine candidates. The RBD dimers previously described in the literature depend on constructions containing an unpaired cysteine residue (corresponding to Cyssss) to establish an artificial intermolecular disulfide bond that is absent from the native structure of protein S. It is highly unlikely that each individual chain in these dimers adopts a spatial orientation relative to the other monomer similar to that observed in adjacent RBDs in protein S trimers, as the distance between adjacent chains of the spike is larger than the size of the Lys₅₂₉-Cys₅₃₈ hinge loop even if the latter were folded in a fully extended conformation. Additionally, these disulfide-bonded dimers do not exhibit the 10 nm spacing necessary to fully engage an ACE2 dimer. A similar situation takes place in the case of the RBD trimers known in the state of the art, which are fusion proteins where the RBD moiety is joined to trimer-forming helical *coiled-coil* segments. The disadvantage of this strategy is that the resulting trimers exhibit a radial arrangement where the N- and C-terminal ends of the RBD segments are held close to the coiled-coil region and hence, also close to the N- and C-terminal ends of adjacent RBD chains, unlike in protein S trimers where there is a substantial separation between them. These trimers, therefore, also fail to reproduce the stereochemical characteristics required to fully engage an ACE2 dimer.

The design of the chimeric proteins described herein not only affords a higher stability through the formation of a network of additional interactions, but provides highly flexible (d) spacers that confer the RBD segments of the chimeric proteins bound to HBcAg the ability to exhibit a dynamic behavior similar to the "breathing" pattern observed in protein S trimers.

The chimeric proteins disclosed in the present invention also include an extrinsic component, provided by modules (c) and (e). The central element of this component is the hexahistidine sequence of module (c), which can mediate processes of trimerization and/or dimerization via the formation of coordination bonds with transition metals such as Ni and Zn (Figure 2). The affinity reported in the state of the art for this type of interaction lies in the nanomolar range, which would turn this interaction into a highly stabilizing node within the stabilization network of the chimeric RBD proteins in complex with HBcAg.

However, it is not straightforward that a hexahistidine sequence forming coordination complexes with transition metals can be put to such practical use. Not only must the hexahistidine segments be well exposed and accessible if they are to interact, but since the RBD modules are associated to the HBcAg spikes, it becomes necessary to connect modules (c) to the RBD module using spacers that enable the hexahistidine segments to interact mutually in a space within no less than 2 nm from the spikes. Modules (e) are designed to fulfill this role, as they are flexible and rich in serine residues to guarantee the adoption of extended conformations (Figure 2). In the case shown, the (e) residues immediately adjacent to the end of the RBD segment are rich in glycine residues, conferring higher local flexibility to this portion and lowering structural constraints to a minimum. The essential element of the module is the presence of an at least 10-residue long poly serine segment. In this invention, an analysis has been carried out of poly serine-type sequences or serine-rich segments present in proteins whose 3D structure has been determined and their coordinates deposited at PDB. This analysis indicated that a segment of ten consecutive serines guarantees a spacing of 20 Å (Figure 2). As shown by the superposition of poly serine segments, they can adopt a variety of conformations -indicating a certain degree of flexibility- but they also exhibit some unidirectional rigidity, useful for the properties required of the modules (e) disclosed in the present invention. Figures 2C and 2D show an example of trimerization mediated by hexahistidine segments coordinating two Ni atoms.

In the present invention, a network of interactions between the RBD-containing chimeric proteins is established, whose nodes are the (a) modules associated to the HBcAg spikes and the (c) modules trimerized or dimerized through the coordination of transition metal atoms by their hexahistidine segments.

The chimeric proteins of the present invention can form a corona over the HBcAg nanoparticle (Figure 3) that has unique properties, without precedent in the state of the art. This corona mimics essential structural aspects of the RBDs from SARS-CoV-2 virions that are fundamental for the generation of a robust, high-quality neutralizing antibody response and go from the trimeric quaternary structure to the spacing determined by its supramolecular structure and its intrinsic flexibility, owing to the use of flexible spacers. Such a degree of structure-function mimicry has never been reported. Obtaining a corona with such characteristics was possible through the novel design of the interaction network disclosed in the present invention, based on non-covalent interactions that guarantee a high specificity, the ability to self-assemble and ease of manufacturing in practical use, since the process does not require chemical reactions involving the formation of covalent bonds or purification processes to remove leftover reactants or undesired products due to side reactions, etc.

### Example 2. Production of chimeric proteins containing RBD segments

Following the design shown in Example 1, it was decided to produce different proteins containing the RBD segment in which both the exact length of the RBD segment as well as the length and sequence of the spacers between the RBD segment and the HBcAg-binding sequence or the hexahistidine dimerization/trimerization motif were varied, even omitting entirely the HBcAg-binding sequence, the dimerization/trimerization sequence, or both. These variants all maintain the same general organization: in N- to C-terminal direction they have an HBcAg-binding sequence, a first, variable-length spacer, the RBD, a second spacer also of variable length and a dimerization/trimerization sequence (a hexahistidine, in this case).

The features of said variants are shown in Table 1, in N- to C-terminal orientation (RBD residue numbering follows that of GenBank acc. number QHD43416). The HBcAg-binding sequence is omitted in five of the polypeptides shown in Table 1. Likewise, the first spacer is absent in most of the variants shown in the table, and the second spacer is missing in three of the obtained variants.

**Table 1. Characteristics of the polypeptides containing an RBD segment.**

| Name | HBcAg-binding sequence | Spacer 1 | RBD sequence | Spacer 2 | Trimerization sequence | SEQ ID NO: |
|---|---|---|---|---|---|---|
| RBD | - | - | 331-531 | - | - | 1 |
| RBDH | - | - | 331-531 | - | *HHHHHH* | 2 |
| RBDcmycH | - | - | 331-530 | *REQKLISEE DLNSAVD* | *HHHHHH* | 3 |
| CRBDH6-P | NWSFFSNI | GGSSGGS | 331-529 | *GGSGGSSSS SSSSSSIE* | *HHHHHH* | 4 |
| RBDH6-P | - | - | 331-529 | *GGSGGSSSS SSSSSSIE* | *HHHHHH* | 10 |
| CRBDH6-H | WSFFSNI | GGSGG | 328-533 | AAA | *HHHHHH* | 5 |
| RBDH6-H | - | - | 328-533 | AAA | *HHHHHH* | 11 |
| CRBD | NWSFFSNI | - | 331-531 | - | - | 6 |

In order to use an RBD variant a) as compact as possible, and b) with no unpaired cysteines, RBD 331-530 (SEQ ID NO:1) was chosen as the starting vaccine antigen. This variant extends 5 residues towards the N-terminus starting from the first cysteine of the domain and thus includes the two N-glycosylation sites of the N-terminal portion of the domain which, according to data previously obtained with the RBD of SARS-CoV-1, are important for the expression of said domains in microbial hosts (Chen WH, et al. Hum Vaccines Immunother. 2014;10(3):648-58), and extends 4 residues towards the C-terminus starting from the last cysteine of the domain. The choice of C-terminal boundary was based on the existence of several examples in the literature where RBDs with this C-terminus are successfully expressed in microbial hosts (Premkumar L, et al. Sci Immunol. 2020;5(48):1-15; Malladi SK, et al.. bioRxiv [Internet]. 2020 Jan 1).

In order to obtain variants RBD, RBDH, RBDcmycH, CRBDH6-P, RBDH6-P and CRBD, gene synthesis following the method of Agarwal *et al.* (Agarwal KL, et al. (1970), Nature 227, 27-34), starting from solid phase-synthesized oligonucleotides obtained from phosphoramidites (Beaucage SL, Caruthers MH, Deoxynucleoside phosphoramidites- A new class of key intermediates for deoxypolynucleotide synthesis., Tetrahedron Letters, (1981), 22, 1859) which was used to produce DNA fragments flanked by *Sap* I sites that coded for said variants and enabled their insertion into plasmid pPICZalfaA (Invitrogen Corp. USA) in such a way that they were fused, in-phase, to the sequence coding for the *Saccharomyces cerevisiae* alpha mating factor. In the case of variants CRBDH6-H and RBDH6-H, the chemical synthesis method mentioned above was also used to obtain fragments coding for them fused to sequences coding for the signal peptide of the human protein LRP1 (SEQ ID NO: 9), flanked by BamH I and Xba I restriction sites.

In the case of variants RBD, RBDH, RBDcmycH, CRBDH6-P, RBDH6-P and CRBD, vector pPICZalfaA (Invitrogen Corp. USA) was amplified by Polymerase Chain Reaction (PCR) (Saiki, R. K. et al. (1985) 'Enzymatic amplification of beta-globin genomic sequences and restriction site analysis for diagnosis of sickle cell anemia', Science, 230(4732), pp. 1350-1354) with the Phusion^{®} High Fidelity PCR kit (New England Biolabs Inc., USA) and primers (**Error! Reference source not found.**7 and **Error! Reference source not found.**8), which introduce Sap I restriction enzyme sites complementary to those already present in the synthetic fragments described above. After digestion with Sap I (New England Biolabs Inc., USA) under the conditions recommended by the manufacturer of the PCR-amplified vector as well as of the fragments coding for variants RBD, RBDH, RBDcmycH, CRBDH6-P, RBDH6-P and CRBD, each digested fragment was enzymatically ligated to pPICZalfaA/Sap I, using T4 DNA ligase (Promega Corp. USA), also under the conditions recommended by the manufacturer.

In the case of variants CRBDH6-H and RBDH6-H, vector pcDNA3 (Invitrogen Corp., USA) was digested with the enzymes BamH I and Xba I (New England Biolabs Inc., USA,) and ligated separately with T4 DNA ligase (Promega Corp. USA,), under the conditions recommended by the manufacturer, to the synthetic DNA fragments corresponding to these variants, digested identically.

In all cases the resulting reactions were transformed in the *Escherichia coli* strain DH5alfa (Anonymous (1986) 'BRL pUC host: E. coli DH5α competent cells', Focus, 8(2), p. 9) according to Sambrook *et al.* (Sambrook J, Fritsch EF, Maniatis T. Molecular cloning: A laboratory manual. New York, USA: Cold Spring Harbor Laboratory Press; 1989), and after plasmid DNA was purified from several of the colonies obtained in selective media, said plasmid samples were screened by restriction analysis. The sequence of several of the resulting recombinant plasmids whose restriction pattern matched that expected for a recombinant clone was verified by automatic Sanger sequencing, and for each RBD variant a representative clone whose sequence matched the expected sequence was chosen. The physical and restriction maps of the resulting plasmids are shown in **Error! Reference source not found.**4.

For the production of variants RBD, RBDH, RBDcmycH, CRBDH6-P, RBDH6-P and CRBD, the corresponding plasmids were previously digested with the Pme I restriction enzyme (New England Biolabs Inc., USA) under the conditions indicated by the manufacturer and transformed into strain X-33 of the methylotrophic yeast *P. pastoris* (Higgins, D. R. et al. (1998) 'Small Vectors for Expression Based on Dominant Drug Resistance with Direct Multicopy Selection', in Pichia Protocols. New Jersey: Humana Press, pp. 41-54) through the procedure described by Wu *et al.* (Wu, S. and Letchworth, G. J. (2004), BioTechniques, 36(1), pp. 152-4), selecting the transformants with Zeocin^{™} at 100 µg/mL (Invitrogen Corp., USA). One colony resulting from the transformation of each plasmid was inoculated into 200 mL of BMGY medium (Pichia Expression Kit. A Manual of Methods for Expression of Recombinant Proteins in Pichia pastoris. Version M 011102 25-0043, Invitrogen Corp., USA, 2002) in a 2 L Erlenmeyer flask and cultured at 28°C, 180 rpm to an optical density (OD) of 8 at 600 nm, after which the cells were harvested by centrifugation at 3000 × g, 4°C, 10 min and resuspended into 200 mL of BMMY medium (Pichia Expression Kit. A Manual of Methods for Expression of Recombinant Proteins in Pichia pastoris. Version M 011102 25-0043, Invitrogen Corp., USA, 2002) that were transferred into a 2 L Erlenmeyer flask. The resulting culture was incubated for 72 h at 28°C, 180 rpm, adding methanol to a final concentration of 0.5% (v/v) every 24 hours. Afterward, the culture was centrifuged at 3000 × g, 4°C, 20 min. The supernatant was filtered through a 0.22 µm membrane and equilibrated into 50 mM phosphate buffer pH 8, 300 mM NaCl, 10 mM imidazole by diafiltration.

For the production of variants CRBDH6-H and RBDH6-H, plasmids pcDNA3-CRBDH6-H and pcDNA3-RBDH6-H were transformed, using polyethylenimine (PEI) (Boussif, O. et al. (1995) 'A versatile vector for gene and oligonucleotide transfer into cells in culture and in vivo: polyethylenimine.', Proceedings of the National Academy of Sciences of the United States of America, 92(16), pp. 7297-301) into the HEK293 (Graham, F. L. et al. (1977), Journal of General Virology, 36(1), pp. 59-72) and CHO (Wurm, F., Hacker, D. Nat Biotechnol 29, 718-720 (2011)) cell lines. In both cases the cells were cultured in DMEM medium (ThermoFisher Scientific, USA) with glucose at 4.5 g/L, supplemented with 10% fetal bovine serum (Natocor, USA) and sodium pyruvate at 110 mg/L at 37°C, in 5% CO₂. The cells were grown for 72 h, and the culture supernatants, after filtration through a 0.22 µm membrane, were equilibrated into 50 mM phosphate buffer pH 8, 300 mM NaCl, 10 mM imidazole by diafiltration.

In all cases, the preparations equilibrated into phosphate buffer were purified by ion metal affinity chromatography (IMAC) (Sulkowski, E. (1985) Purification of proteins by IMAC. Trends Biotechnol. 3, 1-7) using Ni-NTA agarose (Qiagen Benelux B.V., The Netherlands) under the conditions recommended by the manufacturer. The resulting preparations were subjected to a final step of reversed phase hydrophobic interaction chromatography, with a ligand density of C4 and particle size of 15 to 20 µm, equilibrating the resin in a solution of trifluoroacetic acid (TFA) at 0.5% (v/v) (solution A) and applying a 40 minutes gradient with a solution of acetonitrile with 1% (v/v) TFA (solution B). After removing the acetonitrile and conditioning the final samples in 20 mM Tris-HCl buffer pH 7.4, the RBD variants were obtained at a concentration higher than or equal to 0.7 mg/mL and a purity of 98-99%.

### Example 3. Formation of hybrid nanoparticles composed of HBcAg and chimeric CRBDH6 proteins containing an RBD segment

With the objective of monitoring the binding of the chimeric CRBDH6 proteins disclosed in the present invention to the HBcAg nanoparticle, a study employing gel filtration chromatography with an analytic Superdex 75 10/30 column was set up, using PBS as running buffer at a flow of 0.9 ml/min and detection by absorbance at 214 nm. The column was first calibrated with standards from the low molecular calibration kit of General Electric^{™} (see inset in Figure 5), demonstrating that under these running conditions it is possible to achieve optimal separation -according to molecular weight-of monomers, dimers and trimers or higher order aggregates of said CRBDH6 chimeric proteins.

A first experiment analyzed three samples: protein CRBDH6-CHO (expressed in CHO cells), the HBcAg nanoparticle, and a mixture thereof. All samples were equilibrated in phosphate buffer pH 7.4, at a protein concentration of 4 µM for CRBDH6-CHO and 20 µM for HBcAg -thus, the latter molecule was present at a five-fold molar excess when both proteins were mixed. Before their analysis by chromatography, the three samples were incubated for 2 hours at 37°C. As shown in Figure 5, most of CRBDH6-CHO is present as a monomeric species, although it is possible to detect the presence of dimers and trimers (or multimers). The appearance of species size larger than the monomer is not caused by the incubation at 37°C, since it can also be observed at room temperature (24°C, not shown). An unavoidable characteristic of gel filtration studies is that the samples get diluted during the run, and in this case the dilution factor calculated for the monomer peak of the RBD protein is 15. This indicates that the final effective concentration of the protein is actually lower than 267 nM, and that the association constant of the protein is high enough for the existence of dimers and higher-order species at the submicromolar range to be detectable. HBcAg eluted within the exclusion volume of the column, which is consistent with the self-assembly of this protein into nanoparticles larger than 20 nm composed of hundreds of monomers (240 chains, in the case of T4 icosahedral symmetry). The chromatogram corresponding to the complex (HBcAg nanoparticle and CRBDH6-CHO) indicated that the chimeric protein CRBDH6-CHO binds the HBcAg nanoparticle and a considerable fraction remains associated despite the dissociation that must necessarily take place upon dilution of the sample during the run, suggesting an association constant in the submicromolar range. The figure also shows that the relative decrease of the dimer signal is larger than that of the monomer, consistent with the principle of intrinsic stabilization followed during the design of the hybrid nanoparticles disclosed by the present invention.

Next, the chimeric protein CRBDH6-P, produced in *P. pastoris,* and its association to the HBcAg nanoparticle at 37°C was also studied. In this case protein concentration was 3.4 µM for CRBDH6-P and 20 µM for HBcAg. As can be observed in Figure 6A, the main peak of protein CRBDH6-P has a retention time that corresponds to the dimer of protein CRBDH6-H, although this information is not sufficient to conclude that the signal corresponds, indeed, to a dimer.

The study performed by SDS-PAGE on chimeric proteins CRBDH6-P and CRBDH6-H (Figure 8) demonstrated that protein CRBDH6-P has a relative electrophoretic migration significantly smaller than CRBDH6-H. This difference is attributable to a larger extent of N-glycosylation in CRBDH6-P, as the relative electrophoretic migration of both proteins is similar upon N-deglycosylation. Regardless of any uncertainties about the migration of protein CRBDH6-P, the chromatographic profile clearly shows the presence of minor species of higher molecular weight, corroborating the existence of these species at effective concentrations down to the submicromolar range. The chromatographic profile of the HBcAg-CRBDH6-P complex indicates that protein CRBDH6-P is binding to HBcAg, as the area under the curve of the CRBDH6-P peak decreases and that of the HBcAg nanoparticle increases quantitatively.

The complex also forms at a temperature of 50°C, as shown in Figure 6B. The fraction of CRBDH6-P protein forming complexes with HBcAg is even larger in this case - around 50%, indicating that the equilibrium constant K_{d} falls into the submicromolar range (200 nM, approximately) if it is assumed that the starting concentration of the protein (3.4 µM) decreases 15-fold by dilution. According to this analysis, the "affinity" of the interaction is higher than expected and, therefore, not foreseen, since the previous art-reported value of the affinity of the HBcAg binding peptide, or module (d) in the present invention, is only 12 µM. This indicates that the additional stabilization (intrinsic, in this case) provided by the design of the chimeric proteins guarantees at least a 50-fold increase of the binding affinity to the HBcAg nanoparticles.

Figure 6C shows the result of an experiment analogous to the two former experiments but incubating the samples for 2 hours at 18°C. In this case there is no detectable complex formation, indicating that temperature plays a critical role in the interaction between CRBDH6-P and the HBcAg nanoparticle. Also, there was no detectable binding to HBcAg of protein CRBD (Figure 7), which has an N-terminal module (b) to interact with the spikes of HBcAg nanoparticle but does not have module (d), the flexible spacer described in the present invention. This result indicates that the sole presence of a WSFFSNI sequence, which exhibits HBcAg-binding activity as a synthetic peptide, is not sufficient to promote complex formation with the HBcAg nanoparticle in the case of fusion proteins. The latter case requires an adequate design involving spacers and a set of novel elements such as those disclosed in the present invention.

As mentioned above, the data from these experiments demonstrates that temperature is a key factor in the formation of HBcAg-CRBDH6-P hybrid nanoparticles, since no detectable association exists when both proteins are incubated for 2 hours at 18°C whereas an HBcAg-associated fraction of CRBDH6-P becomes clearly detectable at 37°C and increases further at 50°C. The design of the chimeric proteins disclosed in the present invention comprises the flexible spacers at both ends of the RBD domain, which has specially designed stereochemical features introduced to favor the formation and stability of the complexes. Module (d), connecting the RBD module with the HBcAg-binding module (b), is rich in glycine and contains several serine residues to increase its solubility. Higher temperatures favor the movement of this spacer, increasing its flexibility and augmenting the accessibility of the HBcAg-binding module and its availability for interaction with the nanoparticles. In the same vein, it should be taken into account that the HBcAg-binding sequence contains aromatic residues (one tryptophan and two phenylalanines) and polar residues (two serines and to asparagines) that favor protein-glycan interactions. Considering that these chimeric proteins are expressed in a highly glycosylated state, there is a high probability that this segment interacts with nearby sugars in the same polypeptide chain, getting trapped into local energy minima that impose a requirement for thermal energy to overcome their activation energy barriers if the sequence is to interact with HBcAg. Another difference observed at 50°C is that the fraction of self-aggregated CRBDH6-P decreases with respect to the fraction observed at 37°C. Probably, segments (d) and (b) contribute to the formation of protein dimers/trimers/oligomers of low stability that are "dissolved" via thermal movement.

### The binding of CRBDH6-P and CRBDH6-H to HBcAg to form hybrid nanoparticles blocks the interaction of the latter with anti-HBcAg antibodies. Role of the hexahistidine modules (c) and metal coordination for dimerization/trimerization on the extrinsic stabilization of the complexes.

A competition ELISA was also set up to study the association of protein CRBDH6-P with HBcAg. First, a 96-well microtiter plate was coated with a purified specific polyclonal antibody, raised in rabbits (purity higher than 95%). The polyclonal antibody was diluted with coating buffer (carbonate/bicarbonate 0.1 M, pH 9.6) to a final concentration of 0.5 µg/mL (100 µL per well). After incubation for 2 hours at 37°C, the microtiter plate was washed twice with washing buffer (PBS/Tween-20 at 0.05%) and then 200 µL of blocking buffer (PBS/Tween-20 at 0.05%/skimmed milk at 0.05%) were added per well, incubating the plate for 1 hour at 25°C and then washing it twice as described above. In parallel, proteins comprising the RBD were prepared at a range of concentrations (from 15 µM to 0.12 µM) in PBS/Tween-20 at 0.05%, mixed with a fixed concentration of HBcAg (50 ng/mL) and incubated for 2 hours at 37°C. Then, 100 µL of each RBD-HBcAg mixture were loaded onto separate wells, and the microtiter plate was incubated for 1 hour at 25°C and washed five times as described above. Afterward, 80 µL were loaded per well of a polyclonal anti-HBcAg antibody conjugated to peroxidase (diluted 1/5000 in PBS/Tween-20 at 0.05%) and the plate was incubated for 1 hour at 25°C, followed by washes and the addition (100 µL per well) of TMB, a chromogenic peroxidase substrate, together with H₂O₂. Finally, H₂SO₄ was added to the plate to stop the reaction, recording absorbance at 450 on a microplate reader (Thermo-Scientific, Finland).

Five proteins were studied: CRBDH6-CHO, CRBDH6-P and the controls RBD, CRBD and RBDcmycH, described in Example 2. Variants RBD and RBDcmycH do not have modules (b) and (d) and therefore are devoid of HBcAg-binding sequences, while variant CRBD has the HBcAg-binding module (b) but without the spacer in module (d). As shown in Figure 9, when proteins CRBDH6-CHO and CRBDH6-P associate with HBcAg to form hybrid nanoparticles, they block the interaction of the anti-HBcAg polyclonal antibody with HBcAg in a dose-dependent manner, exhibiting an IC50 in the low micromolar range. The reason why this inhibition is so effective is twofold: 1) module (b) binds a patch in the HBcAg spike that constitutes the immunodominant epitope of this antigen, against which, by definition, most antibodies in the polyclonal anti-HBcAg preparation are directed, and 2) proteins CRBDH6-CHO and CRBDH6-P are physically placed above the spikes of the HBcAg nanoparticle, creating a corona that blocks effectively any anti-HBcAg antibody.

In this assay, under the same experimental conditions, protein CRBDH6-CHO inhibited the binding of anti-HBcAg antibodies more effectively than protein CRBDH6-P. The latter only blocks the polyclonal antibodies partially, probably due to the larger size of the carbohydrate moieties on its N-glycosylation sites, which may thus decrease the number of available interaction sites through steric hindrance. However, if Ni is added to CRBDH6-P, the occupation of binding sites increases to levels similar to those of CRBDH6-CHO, and the IC50 as a consequence falls into the submicromolar range. This observation demonstrates that the design of the combination of modules (c) and (e) increases the (extrinsic) stability of the complexes formed by these proteins with HBcAg through the formation of C-RBD-H dimers/trimers via the coordination bonds of the hexahistidine segments.

Figure 9 also demonstrates that protein CRBD does not inhibit the binding of anti-HBcAg antibodies at the assayed concentrations. Therefore, the presence of an HBcAg binding module (b) is not sufficient, in and of itself, to promote the efficient binding to HBcAg nanoparticles. This result underscores how the properties of the chimeric proteins disclosed in the present invention are not foreseen from previous art demonstrating the ability of peptides with the WSFFSNI sequence to bind HBcAg. In protein CRBD module (b) is directly fused to the RBD module without a connecting spacer and is probably sterically occluded by intramolecular interactions that hinder its interaction with its binding site on the HBcAg spike, and/or the affinity of the binding is affected by unfavorable constraints of the same character. The present invention includes the design of module (b), a glycine-rich spacer that gives it the necessary flexibility and protuberance to achieve an efficient binding.

### Example 4. ACE2-binding assays of the chimeric CRBDH6-H and CRBDH6-P proteins and of hybrid nanoparticles containing HBcAg and said chimeric proteins.

These assays were intended to further characterize the interaction between some variants of the chimeric proteins of the present invention and the ACE2 receptor, immobilized on the surface of a chip. The following proteins were used in the assay: 1) chimeric ACE2-mFc protein (immobilized ligand) produced in a mammalian cell line (HEK293), at a concentration of 0.93 mg/mL in phosphate buffer pH 7.2; 2) chimeric proteins CRBDH6-H and CRBDH6-P; 3) negative controls (EGFhr and DIII(DV)-H6), produced in recombinant *S*. *cerevisiae* and *E*. *coli* strains, respectively.

BIACORE analyses: The analyses were performed in a BIACORE X (General Electric, USA) biosensor, using PBS (Sigma-Aldrich, USA) as running buffer. The immobilization was performed at 25°C, at a flow of 5 µL/min. The surface of a CM5 chip was activated by applying 35 µL of a 0.2 mol/L solution of 1-ethyl-3-(3dimethylaminopropyl) carbodiimide (Sigma-Aldrich, USA) and 50 mmol/L N-hydroxysuccinimide (Sigma-Aldrich, USA) in water, followed by a solution of 30 µg/mL of ACE2-mFc in 10 mmol/L CHsCOONa, pH 5.0. Afterwards, 35 µL were loaded of 1 mol/L ethanolamine, pH 8.0 to block any remaining free activated NH₂ groups. The channel employed as a negative control only received the activation and blocking injections at the same flow and with the same volume. The samples were loaded at a flow of 10 µL/min for 120 s, followed by running buffer, while registering the dissociation, for another 120 s. The surface was regenerated by loading 5 µL of NaOH 10 mM in water. For the analysis of binding kinetics each sample was applied in serial dilutions going from 2 µM to 0.015 µM. Each experiment was repeated twice, with similar results each time. The calculation of the constants used at least 5 curves from different concentrations of each protein. The data were processed with the BIAevaluation V4.1 software package (General Electric^{™}, USA), determining the association (ka) and dissociation (kd) constants as well as the affinity constant (KD) by fitting to a Langmuir 1:1 model. The model was selected based on previously reported BIACORE analyses in the literature for this ligand-receptor pair in a similar experimental format (Wang Q, et al. Cell (2020), 181: 894-904; Jun Lan, et al. Structure of the SARS-CoV-2 spike receptor-binding domain bound to the ACE2 receptor. Nature (2020), 581: 215-220).

The results presented here correspond to determinations for proteins CRBDH6-H and CRBDH6-P as well as for control proteins (analytes in solution) in an experimental format in which the ACE2-mFc receptor is immobilized on the surface of the chip. The sensorgrams for the determinations of each protein, as well as the curves obtained by fitting to a Langmuir 1:1 model (included in the chart), are presented in Figure 10. The samples were studied dissolved into 17 mM citrate/66 mM phosphate buffer at pH 6.2. Substantial variations of the signal were detected in the evaluated range of concentrations (Figure 10), enabling the calculation of association and dissociation rate constants as well as the dissociation constant for this interaction (Table 2). In contrast, the proteins used as negative controls yielded only near-baseline signals.

**Table 2. Rmax values, association and dissociation rate constants (ka and kd), and equilibrium dissociation constant (KD) obtained for each of the samples analyzed in the BIACORE X biosensor.**

| **Sample** | **Rmax (RU)** | **kₐ (1/M·s)** | **k_{d} (1/s)** | **K_{D} (M)** |
|---|---|---|---|---|
| CRBDH6-P | 20.6 | 1.08 × 10⁵ | 2.79 × 10⁻⁶ | 2.57 × 10⁻¹¹ |
| CRBDH6-H | 26.0 | 5.07 × 10⁴ | 7.79 × 10⁻⁷ | 1.54 × 10⁻¹¹ |
| EGFₕᵣ | ND | ND | ND | ND |
| DIII(DV)-H6 | ND | ND | ND | ND |

| | | | | |
|---|---|---|---|---|
| ND: Not determined | | | | |

The response was dependent on the concentration of the analyte in solution and saturable, indicating the presence of a specific ligand-receptor interaction. The association rates were in the order of 10⁵ M⁻¹·s⁻¹, reaching equilibrium at 25 to 30 s in the association phase. The dissociation rate constant had a value of approximately 10⁻⁶ s⁻¹, which is three orders of magnitude slower than what the literature reports for determinations in BIACORE biosensors of the parameters of the RBD-ACE2 interaction. Therefore, the resulting KD fell into the low nanomolar range, close to picomolar (10⁻¹¹ M). In this case the value of the determined affinity constant is closer to *in silico* predictions than to previously reported experimental figures. The result suggests that the affinity of the chimeric RBD-containing proteins for the ACE2 receptor is high, regardless of the expression host used for their production. Both chimeric proteins -CRBDH6-P and CRBDH6-H- exhibited high affinity for the ACE2 receptor immobilized on the chip (K_{D} = 2.57 × 10⁻¹¹ and K_{D} = 1.54 × 10⁻¹¹ M, respectively).

Next, the same experimental setup was used to study the binding of hybrid HBcAg-CRBDH6-H nanoparticles to the ACE2 receptor. The sensorgrams of this protein in citrate/phosphate buffer pH 6.2 are shown in Figure 11. The estimated equilibrium constant was KD = 2.94 × 10⁻¹¹ M (Chi2 = 9.75). Hence, as can be observed both from the shape of the curves and the estimated KD values, the association of the chimeric CRBDH6-H protein with HBcAg does not affect the affinity of the RBD-ACE2 interaction in this system.

### Example 5. Evaluation of the immune response in mice immunized parenterally with two variants of recombinant proteins from Pichia pastoris containing the RBD

A previous Example of Realization described the production, via recombinant strains of the methylotrophic yeast *Pichia pastoris,* of two variants of recombinant proteins containing the RBD (Table 1) that were obtained at > 90% purity with very low pyrogen levels. These two variants were: 1. RBD, comprising the RBD from SARS-CoV-2 without additional modules and 2. CRBDH6-P. The chimeric protein CRBDH6-P can be trimerized through the chelation of metal ions (e.g. Ni²⁺).

In order to study the generation of an anti-RBD humoral immune response by these proteins, ten 6-8 weeks old female Balb/c mice (CENPALAB, Cuba) were immunized intramuscularly (i.m.) with them. The immunization followed a 0-14 schedule, and the immunogens were prepared in a total volume of 100 µL and adjuvanted with 1 mg/mL of aluminum hydroxide (AIOOH) (Superfos Biosector A/S, Vedbaek, Denmark). The experimental groups were: 1. Placebo, 2. RBD-P (20 µg) and 3. CRBDH6-P (20 µg) (the amounts between parentheses represent the dose per animal). The placebo (group G1) was prepared as all the other immunogens but omitting the antigen.

Blood samples were collected from the retro-orbital plexus and centrifuged at 7,830 × g for 10 min. The sera were stored at -20°C until their evaluation.

The specific anti-RBD IgG response was measured by ELISA. Ninety-six well microtiter plates (High-binding^{™}, Costar, Austin, TX, USA) were coated with 100 µL/well of RBDhFc (CIM, Cuba) at 3 µg/mL in coating buffer (carbonate/bicarbonate pH 9.6) and incubated 1 h at 37°C. Next, the plates were blocked with 2% skimmed milk in PBS for 1 h at 37°C and then incubated with serum samples in dilution buffer (1% skimmed milk and 1% Tween-20 in PBS) for 1 h at 37°C. Afterward, the plates were incubated with goat antibodies against total mouse IgG conjugated to peroxidase (Sigma, Milwaukee, WI, USA) at a dilution of 1: 5 000 for 1 hour at 37°C and finally, signal was developed for 10 min. using substrate solution. The absorbance at 492 nm was recorded using a plate reader (SUMA model PR-621, CIE, Cuba). Each step was separated from the following step by at least five washes. The detection of IgA in nasopharyngeal washes was performed with the same procedure, but the samples were used undiluted and using instead a conjugate of goat anti-mouse IgA antibodies (Sigma, Milwaukee, WI, USA) at a 1: 5000 dilution.

IgGₜₒₜₐₗ titers were calculated by interpolating the log of the absorbance at a fixed serum dilution into a model obtained by linear regression of the log of the dilution vs the log of absorbance at 492 nm of a standard curve of a previously titered serum. Then, the antilog of the result of the interpolation was calculated and multiplied by the dilution of the sample. For the interpolation, the highest dilution of the sample still yielding a signal higher than 2 times the absorbance of the negative control (a homogeneous mix of non-immunized animal sera at 1:100 dilution) was chosen.

The neutralization assay was performed as follows. First, a 96-well cell culture plate (COSTAR, USA) was seeded with 20,000 cells/well in DMEM medium supplemented with 5% fetal calf serum, 25 mM glutamine, 80 µg/ml gentamycin and 2 µg/mL bicarbonate. After a 24 hour incubation at 37°C in a 5% CO₂ atmosphere at 95% relative humidity, the supernatants were discarded and the plate received serial dilutions of the test sera (e.g. 1:2 dilutions from a starting 1:20 dilution) that had previously been incubated for 1 hour at 37°C with a fixed, previously determined number of viruses that induces 100% of rupture in a Vero cell line monolayer. At the fourth day, Neutral Red at 0.02% in SSTF was added, the plate was incubated for one additional hour at 25±3°C, washed with SSTF, and was added lysis solution (50% ethanol, 1% glacial acetic acid), followed by a 15-minute incubation at 25±3°C. Finally, optical density at 540 nm was measured and recorded. Neutralization titers were calculated as the highest dilution of the evaluated serum whose optical density value was higher than the threshold, wherein said threshold was calculated as the mean of the optical density of the cell control wells, divided by 2.

Prism 8.4.3 (GraphPad Software, San Diego, USA) was used to compute descriptive statistics and perform group comparisons. The values were transformed into logarithms and checked for normality before comparing anti-RBD titers. Negative sera were assigned an arbitrary titer of 10. Data normality was verified with the D'Agostino and Pearson test, and Mann-Whitney's test was employed for comparisons between groups. The neutralization results were compared with Fisher's exact test. The threshold for statistical significance was p < 0.05.

Ten days after the second dose, IgG titers exhibited an ascending trend in the animals immunized with CRBDH6-P with respect to those immunized with RBD, but statistical signification was not reached (Figure 12). A neutralization assay was employed to verify the functionality of the generated anti-RBD antibodies. Table 3 shows the results obtained 10 days after the second dose. Frequency analysis evidenced that in the group of mice immunized with the chimeric protein CRBDH6-P the proportion of individuals whose sera exhibited neutralization titers equal to or higher than 1:320 was greater than in the group immunized with RBD-P (*p*=*0.0031*, Fisher's exact test).

**Table 3. In vitro neutralization titers of individual serum samples obtained 10 days after the second dose.**

| **Serum/Animal** | **Placebo** | **RBD** | **CRBDH6-P** |
|---|---|---|---|
| 1 | <20 | 80 | 160 |
| 2 | <20 | 160 | 320 |
| 3 | <20 | 40 | 1280 |
| 4 | <20 | 160 | 1280 |
| 5 | <20 | 80 | 640 |
| 6 | <20 | 160 | 160 |
| 7 | <20 | 40 | 640 |
| 8 | <20 | 20 | 80 |
| 9 | <20 | 160 | 640 |
| 10 | <20 | 40 | 1280 |

| | | | |
|---|---|---|---|
| The data correspond to the highest dilution still exhibiting detectable neutralization. | | | |

Essentially, these results indicate that the trimerization that characterizes the chimeric CRBDH6-P protein generates an antibody response qualitatively superior to that obtained with the recombinant RBD protein in monomeric form, evidenced in higher neutralization titers. In that sense, it can be concluded that the chimeric CRBDH6 proteins in their trimeric form (i.e. CRBDH6-P) mimics more accurately the functional epitopes of the RBD as presented by the SARS-CoV-2 virion.

### Example 6. Evaluation of the immune response of mice immunized parenterally with different variants of recombinant chimeric CRBDH6 protein and hybrid HBcAg-CRBDH6 nanoparticles

Example 5 corroborated that immunization with recombinant CRBDH6, which can form trimers, generates a serum immune response with higher neutralizing titers than that obtained with the monomeric recombinant RBD protein. The present example examines whether the association of the HBcAg nanoparticle and the recombinant protein CRBDH6-P can increase the immunogenicity of these preparations and/or the neutralizing titers of the sera from animals immunized with these preparations. In addition to CRBDH6-P, the example uses the chimeric protein CRBDH6-H, expressed in the human cell line HEK-293, as well as other proteins employed as experimental controls.

Once the different variants of the chimeric protein CRBDH6 were expressed by recombinant means in different hosts and obtained with purity higher than 90% and very low levels of pyrogens, their immunogenicity was evaluated in mice. A total of four protein variants, expressed in two different hosts (the methylotrophic yeast P. *pastoris* and the kidney-derived human cell line HEK-293) were tested. These were: 1. CRBDH6-P; 2. CRBDH6-H; 3. RBDH6-P, and 4. RBDH6-H.

With the purpose of studying the generation of an anti-RBD humoral response after inoculation via a parenteral route, 10 female 6-8 weeks old Balb/c mice (CENPALAB, Cuba) were inoculated using the intramuscular route (i.m.). A 0-14 day immunization schedule was used, employing a dose volume of 100 µL and adjuvanting the antigens with 1 mg/mL of aluminum hydroxide (AIOOH) (Superfos Biosector A/S, Vedbaek, Denmark). Each experimental group received a different immunogen. These were: 1. Placebo, 2. HBcAg (negative control group), 3. CRBDH6-P (20 µg), 4. CRBDH6-H (20 µg), 5. HBcAg (5 µg)-CRBDH6-P (20 µg), 6. HBcAg (5 µg)-CRBDH6-H (20 µg), 7. RBDH6-P (20 µg) + HBcAg (5 µg) and 8. RBDH6-H (20 µg) + HBcAg (5 µg). The numbers in parentheses represent the dose per animal. The placebo was prepared following the same procedure applied for the other immunogens but omitting the antigens. Recombinant proteins CRBDH6-P, CRBDH6-H, RBDH6-P and RBDH6-H were dissolved as described in the preceding example. In groups G5 and G6, CRBDH6-P and CRBDH6-H were previously mixed with HBcAg nanoparticles before adjuvanting with AIOOH. The mass excess of protein CRBDH6 relative to HBcAg that was used in these cases implies that all binding sites on the HBcAg nanoparticle will be occupied and therefore these samples did contain a fraction of free chimeric protein and another fraction of hybrid HBcAg-CRBDH6 nanoparticle. In groups G7 and G8 there is no association between the RBD variant and the HBcAg nanoparticle, owing to the absence of an HBcAg binding sequence in the recombinant proteins RBDH6-P and RBDH6-H. Blood samples were drawn from the retro-orbital plexus, and the sera were obtained as described in Example 5. Likewise, the specific anti-RBD IgG response was measured by ELISA, as described in Example 5.

The assay for inhibition of the RBD-ACE2 was performed up as follows. Ninety-six well microtiter plates (High binding^{™}, Costar, USA) were coated with 50 µL/well of ACE2-mFc (CIM, Cuba) at 5 µg/mL in coating buffer (carbonate-bicarbonate solution, pH 9.6) at 4°C for 16 h in a wet chamber. Then, the wells were blocked with 300 µL of a solution of 3% skimmed milk in PBS for 1 h at 37°C. Next, 100 µL/well were added to the plates of a solution where the test sera, diluted 1:200 in 0.2% skimmed milk/Tween-20 at 0.05% in PBS, had been pre-incubated for 1 h at 37°C with RBDhFc (CIM, Cuba) at 20 ng/mL. After an incubation of 1 h and 30 min at 37°C, a conjugate of goat polyclonal antibodies against human IgG Fc and peroxidase, diluted 1:10,000 in 0.2% skimmed milk/Tween-20 0.05% in PBS, was added to the wells and further incubated for 1 h at 37°C. Signals were developed by incubation with 100 µL/well of a TMB solution (Sigma-Aldrich, USA) for 10 min, stopping the reactions with 50 µL of 3 mol/L H₂SO₄. Absorbance at 492 nm was, finally, read and recorded in a plate reader (CLARIOstar, BMG LABTECH, USA). At least 5 washes were performed between every step. Percentage inhibition was estimated as the quotient of the absorbance in wells receiving the sera and the absorbance in wells without test sera (i.e. where the RBD-ACE2 interaction was maximal), expressed as a percentage. The *in vitro* neutralization assay was performed as described in Example 5.

Prism version 8.4.3 (GraphPad Software, San Diego, CA, USA) was used to compute descriptive statistics and perform group comparisons. For the comparison of anti-RBD titers, the values were transformed logarithmically before verifying their normality. The negative sera were assigned an arbitrary titer of 10. Data normality was checked with D'Agostino and Pearson's test and groups were compared by ANOVA, followed by Tukey's as post-test. An ANOVA with Welch's correction was used to compare RBD-ACE2 inhibition percentages, followed by Dunnett's as post-test. The threshold for statistical significance was set at *p* < *0.05.*

Ten days after the second dose there was a statistically significant increase of IgG titers in the sera of mice immunized with immunogens containing hybrid HBcAg-CRBDH6 nanoparticles (G5 and G6), compared to control groups where the different variants of chimeric CRBDH6 proteins were not inoculated together with HBcAg (G3 and G4) or groups where both proteins were present but not forming hybrid nanoparticles (G7 and G8) (*p* <*0.0001*, ANOVA) (Figure 13).

The functionality of the generated anti-RBD antibodies was verified with an assay for the inhibition by said antibodies of the binding between the RBD protein and its receptor, ACE2. Figure 14 shows the results obtained 10 days after the second dose. As expected, the results of the comparisons between the different experimental groups mirrored those previously obtained when comparing IgG titers: groups receiving hybrid HBcAg-CRBDH6 nanoparticles (G5 and G6) had higher inhibition percentages (*p <0.0001*, ANOVA with Welch's correction). It should be pointed out that there is a correlation between the inhibition capacity of polyclonal sera and *in vitro* virus neutralization assays (Zang J, et al. Cell Discov 2020, 6:61). Therefore, this inhibition assay may be considered a surrogate for the neutralizing capacity of the test sera.

Despite the correlation between inhibition and neutralization assays, a further set of experiments was set up where the sera from groups immunized with recombinant protein variants produced in *P. pastoris* (G3, G5 and G7) and their controls (G1 and G2) were examined by *in vitro* neutralization of SARS-CoV-2 infection (Table 4). As can be observed, and in correspondence with the data derived from the inhibition assays, the group inoculated with the immunogen containing hybrid HBcAg-CRBDH6-P nanoparticles (G5) exhibited a larger proportion of sera with neutralizing titers ≥ 640 than the remaining groups (*p*=*0.0351*, Fisher's test).

**Table 4. In vitro neutralization by individual sera obtained 10 days after 2 doses**

| **Sera/ Animals** | **Placebo** | **HBcAg** | **CRBDH6-P** | **CRBDH6-P _HBcAg** | **RBDH6-P + HBcAg** |
|---|---|---|---|---|---|
| 1 | <20 | <20 | 320 | 1280 | 320 |
| 2 | <20 | <20 | 320 | 2560 | 160 |
| 3 | <20 | <20 | 640 | 640 | 160 |
| 4 | <20 | <20 | 320 | 1280 | 320 |
| 5 | <20 | <20 | 80 | 2560 | 160 |
| 6 | <20 | <20 | 1280 | 320 | 640 |
| 7 | <20 | <20 | 320 | 1280 | 320 |
| 8 | <20 | <20 | 80 | 640 | 320 |
| 9 | <20 | <20 | 320 | 640 | 640 |
| 10 | <20 | <20 | 640 | 320 | 80 |

| | | | | | |
|---|---|---|---|---|---|
| The data shown correspond to the highest dilution still exhibiting detectable neutralization. | | | | | |

Analyzed as a whole, the data indicate that the parenteral (i.e. intramuscular) immunization of mice with immunogens containing hybrid HBcAg/CRBDH6 nanoparticles increases anti-RBD specific IgG titers and the neutralization potency of the resulting sera. This immunostimulatory effect takes place only in the presence of hybrid nanoparticles, since animals receiving a mixture of HBcAg with recombinant proteins RBDH6-H or RBDH6-P, which do not associate with the HBcAg nanoparticle, produced clearly inferior immune responses. Also, the immunostimulatory effect observed for these hybrid nanoparticles did not change with the differences in amino acid sequence or post-translational modifications between variants of the chimeric CRBDH6 protein. In this sense, it is important to underline that one variant (CRBDH6-P) was expressed in the methylotrophic yeast *P. pastoris* and the other (CRBDH6-H) in a cell line of human origin, and therefore both proteins exhibit considerable differences regarding their glycosylation patterns.

### Example 7. Evaluation of the immune response of mice immunized mucosally with different variants of the recombinant chimeric protein CRBDH6 and of hybrid HBcAg-CRBDH6 nanoparticles

Example 6 demonstrated that the formation of hybrid HBcAg-CRBDH6 nanoparticles increases even more the resulting anti-RBD immune response as well as the neutralizing capacity of the resulting sera when delivered using the parenteral route. The present example examines the same question when delivering the same antigens via the mucosal (i.e. intranasal) route.

Ten female, 6-8 weeks old Balb/c mice (CENPALAB) were inoculated through the intranasal route (i.n.) in a 0-14 day scheme with a volume of 50 µL per dose, diluting the antigens in citrate buffer, pH 5.2. The groups, according to the immunogen they received, were: 1. Placebo, 2. HBcAg (negative control group), 3. CRBDH6-P (20 µg), 4. CRBDH6-H (20 µg), 5. HBcAg (5 µg)+CRBDH6-P (20 µg), 6. HBcAg (5 µg)-CRBDH6-H (20 µg), 7. RBDH6-P (20 µg) + HBcAg (5 µg) and 8. RBDH6-H (20 µg) + HBcAg (5 µg).

The sera were obtained as described in Example 6. The nasopharyngeal washes were obtained as described by Cho et al. cols. in 2012 (Cho SH, Oh SY, Zhu Z, Lee J, Lane AP (2012). PLoS ONE 7(4): e35114. https://doi.org/10.1371/journal.pone.0035114) and stored at -20°C until assayed. The specific anti-RBD IgG response and the inhibition of the RBD-ACE2 interaction were evaluated as described in Example 6.

Prism version 8.4.3 (GraphPad Software, San Diego, CA, USA) was used to compute descriptive statistics and to perform comparisons between groups. Anti-RBD titers were transformed logarithmically before checking their normality. Negative sera were assigned an arbitrary titer of 10. Data normality was checked using D'Agostino and Pearson's test. Group comparisons were performed with the Kruskal-Wallis test, using Dunn's as post-test. Percent inhibition values of the RBD-ACE2 interaction were compared by ANOVA with Welch's correction, using Dunnett's as post-test. The statistical significance threshold was set at *p* < *0.05.*

Again, the groups inoculated via the i.n. route with immunogens containing hybrid HBcAg-CRBDH6 nanoparticles (G5 and G6) exhibited, 10 days after the last dose, a statistically significant, higher level of anti-RBD IgG titers in serum than their respective control groups where the chimeric CRBDH6 proteins were not inoculated together with HBcAg (G3 and G4) or were not forming hybrid nanoparticles (G7 and G8) (*p*<*0.0001*, Kruskal-Wallis) (Figure 15). Moreover, since the objective of an intranasal inoculation is mainly to elicit an antibody response at the level of the nasopharyngeal mucosa, nasopharyngeal washes performed with 100 µL of standard saline solution (0.9% NaCl) on six animals randomly chosen from each group were also evaluated with an ELISA for the detection of specific anti-RBD IgA (Figure 16). In this case, total, 100% seroconversion was observed only in the groups receiving immunogens that contained hybrid nanoparticles. In addition, mean absorbance in these groups (G5 and G6) was > 3.5-fold higher than in groups G7 y G8, whose seroconversion levels were very low (33%).

The functionality of the anti-RBD serum antibodies generated by the i.n. immunizations was verified with an assay for the inhibition of the interaction between the RBD protein and its receptor, the ACE2 molecule, by said antibodies. Figure 17 shows the results obtained 10 days after the second dose. As expected, the results of the comparisons between groups mirrored those previously obtained with IgG titers. In this sense, the groups immunized with immunogens containing hybrid nanoparticles exhibited the highest values (*p* <*0.0001*, ANOVA with Welch's correction). The other groups did not exhibit statistically significant differences with controls G1 and G2 and can therefore be said to elicit no detectable responses.

When analyzed as a whole, in this case the data also indicate that the immunization of mice through a mucosal route (i.e., intranasal) using immunogens that contain hybrid HBcAg-CRBDH6 nanoparticles induces a statistically significant increase of specific anti-RBD titers in the serum of immunized animals. Furthermore, intranasal inoculation leads to the appearance of IgA anti-RBD antibodies in the nasopharyngeal mucosa of 100% of the animals, which may potentially help contain a viral infection at the level of the mucosa. This improved immunostimulatory effect exhibited by hybrid HBcAg-CRBDH6 nanoparticles disappears when using RBD variants that do not associate with the HBcAg nanoparticle, as these yield clearly inferior immune responses. It is also worth pointing out that this immunostimulatory effect was observed in hybrid nanoparticles obtained either with the CRBDH6-P or the CRBDH6-H chimeric proteins, despite their differing glycosylation patterns.

### Example 8. Formation of hybrid HBcAg-CRBDH6 nanoparticles that induce a highly potent neutralizing response. Quaternary and supramolecular structure of chimeric CRBDH6 proteins and hybrid HBcAg-CRBDH6 nanoparticles

A central element in the present invention is the ability of the designed chimeric proteins to self-associate into homo-dimers, trimers and/or multimers that mimic the intramolecular interactions observed in the native structure of the viral protein S trimer. As described in the Description of the Invention and Example 4, modules (b) and (c) contribute, via extrinsic association, to the innate (intrinsic) propensity of RBD domains to associate in a manner homologous to that observed in protein S trimers. The HBcAg-binding segment - module (b) - is rich in aromatic and polar residues that tend to interact with carbohydrates (Banno M et al. Computational Biology and Chemistry, 66, 36-43, 2017) and may therefore contribute to said associations, since the RBD segment contains two glycosylation sites. Module (c) contains a hexahistidine sequence which, in addition to its capacity to form dimer and trimers via the formation of coordination bonds with transition metals, also tends to interact with carbohydrates. Additionally, the HBcAg-binding sequence is a heptapeptide in which four out of seven residues are: one tryptophan, two phenylalanines and one isoleucine, all with side chains that tend to interact well with surfactants and amphiphilic compounds in general -such as polysorbates, fatty acids, phospholipids, etc.- especially at concentrations above their critical micellar concentration (CMC). In this sense, Example 3 of the present invention showed how hybrid nanoparticles formed in the presence of the surfactant Tween-20 at 0.05% are stable enough for bound CRBDH6 to sterically hinder the union of anti-HBcAg antibodies. Module (b) in protein CRBDH6 may associate directly with surfactant micelles -such as those formed by Tween-20- which would support extrinsically the association of RBD domains. On the other hand, modules (c) may mediate the formation of dimers/trimers by establishing metal coordination bonds that further stabilize the quaternary structure of CRBDH6 proteins and/or hybrid HBcAg/CRBDH6 nanoparticles.

The present example provides further physico-chemical, biophysical and biological evidence pertaining the quaternary structure of chimeric CRBDH6 proteins and of hybrid HBcAg-CRBDH6 nanoparticles, illustrating the role of the CRBDH6 modules designed in the present invention and their impact on the structure-function relationship of these proteins relevant for the formation of hybrid nanoparticles that can induce a highly potent immunological response.

An analysis of the chimeric proteins CRBDH6-P and CRBDH6-H by dynamic light scattering (DLS) (ZetaSizer Nano, Malvern Instruments, UK) demonstrated that under physiological conditions (PBS pH 7.4) and in the absence of HBcAg they still exhibit self-association into dimers, trimers, multimers and, to a lesser degree, supramolecular aggregates. Figure 18A shows that protein CRBDH6-P in PBS (0.3X) is mainly monomeric. The mean diameter of the main peak is 4.8 ± 1.2 nm and the hydrodynamic diameter (dₕ) estimated from the coordinates of the monomer is 4.9 nm. Judging from the width of the distribution this species coexists with some dimers whose dₕ is 6.7 nm, and there are also supramolecular aggregates of 33 ± 9 nm y and 176 ± 55 nm (Figure 18A), although these are minor species as shown in the distribution by volume of Figure 18B. Protein CRBDH6, expressed in CHO cells, exhibits a similar behavior, although the sizes of the complexes are somewhat reduced, probably owing to the relatively smaller size of the attached N-glycans (see the electrophoresis in Figure 8).

In the presence of PBS pH 7.4 plus Tween-20 0.03% and NiSO₄ 1 mM, protein CRBDH6-P exhibits a main peak of 7 ± 2 nm. The width of the distribution is consistent with the existence of monomers (dₕ = 4.9 nm), dimers (dₕ = 6.7 nm), trimers (dₕ = 7.3 nm), hexamers (dₕ = 8.6 nm) and even dodecamers (dₕ = 11.2 nm). Figure 19F-J shows 3D models of proteins CRBDH6-P and CRBDH6-H adopting different quaternary structures that range from monomers to hexamers and dodecamers.

Figure 18C depicts the formation of the HBcAg-CRBDH6-P hybrid nanoparticle. Pure HBcAg nanoparticles, at an antigen concentration of 12.5 µM in PBS Tween-20 0.03% pH 7.4, exhibit a size of 19.5 ± 5 nm. Upon addition of protein CRBDH6-P at a concentration of 6 µM in PBS Tween-20 0.03% NiSO₄ 1mM pH 7.4, the peaks usually observable in pure CRBDH6-P solutions do not appear, and instead the HBcAg peak shifts to 30 ± 10 nm, which is compatible with the formation of the hybrid nanoparticle described in Figure 3.

Hybrid nanoparticles can also be obtained with protein CRBDH6-H as shown in Figure 18D, which depicts the DLS spectrum corresponding to the distribution, by volume, of protein CRBDH6-H (4.4 ± 1 nm), the HBcAg nanoparticle (24 ± 7.6 nm) and the hybrid HBcAg-CRBDH6-H nanoparticle (26 ± 6.2 nm), dissolved in all cases into 20 mM Tris pH7.4, Tween-20 0.03%, fructose 12% and NiSO₄ 1mM. Hybrid nanoparticles are, as can be observed, larger than pure HBcAg nanoparticles and more homogeneous. Figure 18E presents an analysis of the zeta potential of the same samples shown in Figure 18D: protein CRBDH6-H (-8.9 ± 7.2 mV), the HBcAg nanoparticle (-11 ± 7.4 mV) and the hybrid nanoparticle (-7.7 ± 3.4 mV). As can be observed, the hybrid nanoparticle is electrostatically more homogeneous and easily distinguishable from protein CRBDH6-H and HBcAg.

These samples were also analyzed by Transmission Electron Microscopy (Figure 19 A-E). They were negatively stained following standard procedures, and photographs were taken in a MIRA3-TESCAN (TESCAN, Czech Republic) Scanning Electron Microscope fitted with a transmission detector, at 30 kV. Particle size distribution was analyzed with the Fiji distribution of the ImageJ software application (ImageJ, NIH, WI, USA). Diameters were compared with a two-tailed Mann-Whitney test (using p<0.05 as statistical significance threshold).

Although the mean diameter of the hybrid HBcAg-CRBDH6-H nanoparticles is only slightly larger than that of pure HBcAg nanoparticles (27.6 nm vs 27.5 nm), their medians are 27.6 nm vs. 15.4 nm respectively, consistent with a larger size for the hybrid nanoparticles. Also, the contour of hybrid nanoparticles is more irregular than that of pure HBcAg nanoparticles (Figura19 A-E). The difference in diameter -hybrid nanoparticles being larger than HBcAg nanoparticles- is statistically significant (p=0.0023). The size of hybrid nanoparticles is more homogeneous (the standard deviation of their diameter is 16.7 nm vs. 21.1 nm for pure HBcAg nanoparticles).

The impact of the presence of metal ions and an amphiphile in the process of formation of the hybrid nanoparticles disclosed in the present invention was also evaluated. Particularly, the stability of the nanoparticles obtained under a variety of conditions was studied, using as stability criteria the lack of reactivity of anti-HBcAg antibodies toward the resulting nanoparticles or, to word it differently, the ability of CRBDH6 proteins to block the interaction of said antibodies with HBcAg nanoparticles. Solutions of CRBDH6-H were prepared at concentrations ranging from 3 × 10⁻⁶ moles/L to 0.047 × 10⁻⁶ moles/L, to which HBcAg was then added at a fixed final concentration of 50 ng/mL. These solutions were prepared in 20 mM Tris-HCl buffer at pH 7.4 containing Tween-20 at 0.01% (v/v). The samples were incubated for 2 hours at 37°C under constant stirring and then received ZnSO₄ at final concentrations of 0, 0.04 and 0.2 × 10⁻³ moles/L, incubating the resulting mixtures for 30 minutes at 37°C, also under constant stirring. Lastly, the samples were analyzed with the competition ELISA previously described in **Example 3.**

Figure 21A illustrates how achieving an efficient association between the chimeric CRBDH6-H protein and HBcAg requires the inclusion of divalent cations such as Zn⁺². Samples without the cation -whether Tween-20 was present or not- did not exhibit a detectable inhibition of the binding of anti-HBcAg antibodies in the competition ELISA. In contrast, when Zn⁺² was included at concentrations ranging from 0.04 to 0.2 × 10⁻³ mole/L, the samples produced an inhibition of 80 to 100% of the binding of anti-HBcAg antibodies. Although these results, as mentioned above, where independent of the inclusion of Tween-20 at 0.01%, the condition yielding the highest inhibition did include both elements -that is, the divalent cation and the amphiphile. This experiment also corroborates that the association of CRBDH6 to HBcAg requires the presence of module (c), as protein RBDH6-H did not inhibit the binding of anti-HBcAg antibodies under any of the assayed conditions.

The formation of HBcAg-CRBDH6 hybrid nanoparticles was also followed by native electrophoresis in horizontal agarose slab gels (Li, C., & Arakawa, T. (2019). Agarose native gel electrophoresis of proteins. International journal of biological macromolecules, 140, 668-671). The relative migration of the analyte in this technique depends on both its mass and its charge, and so the technique enables the simultaneous analysis of both the proteins and the nanoparticles disclosed in the present invention. Reactions for the association of CRBDH6 to HBcAg were performed as described previously for the competition ELISA, but using a fixed Zn²⁺ concentration of 0.2 mM and using molar CRBDH6:HBcAg ratios of 0.5:1, 1:1 and 2:1. The reaction mixtures were incubated at 37°C for two hours under constant stirring, then ZnSO₄ was added and the reactions were incubated for another 30 minutes at 37°C under constant stirring. Identical conditions were used to prepare the CRBDH6-H controls (Figure 21B, lanes 4 to 6) at the same concentrations used for the association (lanes 1 to 3) and the HBcAg control, at the fixed concentration used of 6 × 10⁻⁶ moles/L (lane 7). Lanes 1 to 3 of Figure 21B show the formation of the hybrid nanoparticles, evidenced by the presence of a diffuse band above the HBcAg bands. The figure demonstrates that the hybrid nanoparticles do form at all three assayed molar ratios, although their homogeneity is higher at the CRBDH6-H: HBcAg ratio of 2:1 (lane 1), which yields a more compact and intense band.

The same native agarose gel electrophoresis system was then used to evaluate the formation of hybrid HBcAg-CRBDH6-P-TSAZn nanoparticles. In this case the association reaction was performed at a 1:1 CRBDH6-P:HBcAg molar ratio in 20 mM Tris-HCl buffer at pH 7.4 and 1 M Zn²⁺, and using 0.1 M stearic acid (SA), an amphiphile, instead of Tween-20. We also evaluated the formation of CRBDH6-P-SA nanoparticles, formed by association of protein CRBDH6-P to 0.1 M stearic acid (that is, in the absence of HBcAg). Figure 21C demonstrates the formation of these two nanoparticles, as evidenced by the presence of a diffuse stain above the HBcAg bands in lane 1 (HBcAg-CRBDH6-P-TSAZn) and in lane 2 (CRBDH6/SA).

The formation of multimeric species of proteins CRBDH6-P and CRBDH6-H, as well as of hybrid HBcAg-CRBDH6-P nanoparticles, was also evidenced by using EGS (Pierce, USA), which is a homobifunctional cross-linker that stabilizes aggregated or multimeric protein species as long as there are two free lysine side chains in different molecules separated by a distance of 16.1 Å.

EGS was used at a 50:1 molar excess with respect to the test protein. In these experiments CRBDH6-P and CRBDH6-H were used at 20 µmol/L (Panels A and B) and 6 µmol/L (Panels C and D) in PBS 0.3X (Panels A, C and D) or 1X (Panel B), pH 7.4. The additives in panel C were Tween 0.015%; fructose 12% and NiSO₄ 3 mmol/L. HBcAg was used at a concentration of 12.5 µmol/L. The cross-linking reaction was incubated for 30 min at 25 °C and then stopped by adding 1 M Tris pH 8.0 buffer to a final concentration of 100 mmol/L. The stopped reactions were analyzed by subjecting aliquots thereof to 12.5% SDS-PAGE.

The cross-linking experiments used as a control a purified preparation of gamma interferon (IFNy). This protein folds into an approximately 32 kDa homodimer that has four positions where the lysine residues from one monomer (16 kDa) are at 7 to 13 Å of the lysine residues of the opposing monomer. As can be observed in Figure 20A and B, the addition of EGS to purified preparations of CRBDH6-P and CRBDH6-H stabilizes species whose molecular mass matches that of dimers (H* and P*), trimers (H**, P**) and multimers (Hⁿ* y Pⁿ*). The same family of species is observed whether the reaction proceeds at PBS 1X or 0.3X, indicating that the formation of multimeric species is not induced at a specific ionic strength. It is also observed in the presence of the additives Tween 0.015%, fructose 12% and NiSO₄ 3 mmol/L (Figure 20C).

The incubation of protein HBcAg with EGS yields a major species of approximately 18 kDa (Figure 20D). Two other minor species are also observed, one of 36 kDa compatible with a dimer, and another of a very high molecular weight. The incubation of protein CRBDH6-P with HBcAg reinforced the high molecular weight band while simultaneously reducing the band corresponding to the trimeric CRBDH6-P species. This result evidences the formation of HBcAg-CRBDH6-P complexes.

Next, the immune response induced by hybrid HBcAg-CRBDH6 nanoparticles formed in the presence of amphiphiles and divalent metal cations was evaluated and compared to that induced by chimeric CRBDH6 alone. This evaluation used groups of 10 female Balb/c mice each, 6-8 weeks old (supplied by CENPALAB, Cuba), which were inoculated intramuscularly (i.m.) with the test preparations following a 0-21-42 regime, and bled 14 days after the third dose. The immunogens were inoculated in a volume of 100 µL, adjuvanted with 1.33 mg/mL of AIOOH (Croda, Frederikssund, Denmark).

The groups of animals (G1-6) according to the immunogen were: G1: placebo; G2: HBcAg (negative control group), G3 (CRBDH6-H-PTFNi): CRBDH6-H (7 µg) dissolved in PBS pH 7.4, Tween-20 0.03%, NiSO₄ 1 mM (PTFNi buffer); G4 (HBcAg-CRBDH6-H-PTFNi): hybrid HBcAg/CRBDH6-H nanoparticle prepared with CRBDH6-H (7 µg) and HBcAg (14.4 µg) in PTFNi buffer; G5 (HBcAg-CRBDH6-PTFZn): hybrid HBcAg-CRBDH6-H nanoparticle prepared with CRBDH6-H (7 µg) and HBcAg (14.4 µg) in PBS pH 7.4, Tween-20 0.03%, fructose 12%, ZnSO₄ 1 mM, and finally, G6 (HBcAg-CRBDH6-PTF): hybrid HBcAg-CRBDH6-H nanoparticle prepared with CRBDH6-P (7 µg) and HBcAg (14.4 µg) in PBS pH 7.4, Tween-20 0.03%, fructose 12% (PTF buffer), that is, without Ni⁺² or Zn⁺² (the figures in parentheses are the dose per animal). The placebo used in G1 was PTFNi buffer, prepared as used for the other immunogens. Blood was collected from the retro-orbital plexus, and serum samples were prepared as described in Example 5. Likewise, measurement by ELISA of the specific anti-RBD IgG response and the *in vitro* neutralization assays were performed as described in Example 5. The assay for inhibition of the RBD-ACE2 interaction was performed as described in Example 6. In order to determine IC50, serial 1:2 dilutions were performed down to a dilution of 1: 102400.

The statistical software package Prism 8.4.3 (GraphPad Software, San Diego, CA, USA) was used to compute descriptive statistics and perform group comparisons. The data were transformed logarithmically before verifying normality and comparing anti-RBD titers. Negative sera were arbitrarily assigned a titer of 10. Data normality was verified with D'Agostino and Pearson's test, and group comparisons were performed with the Kruskal-Wallis test, using Dunn's as post-test. IC50 for the inhibition of the ACE2/RBD interaction were calculated by fitting the log data (dilutions) vs absorbance to a variable slope four-parameter curve. Sera whose inhibition percentage at a dilution of 1:100 was lower than 50% were assigned an arbitrary value of 1:50 for comparisons.

Anti-RBD IgG titers for the individuals of each experimental group were determined 14 days after the third dose. As expected, animals from groups G1 (placebo) and G2 (HBcAg) did not develop an anti-RBD IgG response. In order to evaluate the influence on immunogenicity of the different elements of the invention, we compared the anti-RBD IgG titers of the groups immunized with formulations containing this molecule (groups G3-6). As can be observed in Figure 22A, the mice immunized with the hybrid HBcAg-CRBDH6-H-PTFNi (G4) and HBcAg-CRBDH6-H-PTFZn (G5) nanoparticles elicited much higher levels of antibodies (11.2-fold and 22.8-fold higher, respectively) than the group inoculated with CRBDH6-H without HBcAg (G3) (*p*=*0.0051*, G4 vs G3 and *p*<*0.0001*, G5 vs G3). The presence of metal ions boosts immunogenicity significantly, as the mean titers of groups G4 and G5 are 6.6-fold and 13.4-fold higher than that of group G6 (*p*=*0.0137*, G4 vs G6 and *p*<*0.0001*, G5 vs G6).

The results indicate clearly that the hybrid nanoparticles containing metal ions (the most stable) are the ones eliciting the highest levels of anti-RBD antibodies, and that the nanoparticles obtained in the absence of the metal behave similarly to the chimeric CRBDH6-H protein alone, dissolved into PTFNi buffer.

Next, the quality -functionality- of the antibodies elicited by the different immunogens was studied. In this case, IC50 values for the inhibition of the binding of RBD to the ACE2 receptor were derived from each individual of the groups inoculated with CRBDH6-H (G3), hybrid nanoparticles incorporating Ni⁺² (G4) or Zn⁺² (G5), and nanoparticles formed in the absence of a metal ion (G6) (Fig. 22B). A panel of 10 convalescent sera was also analyzed as a reference.

The antibody response exhibiting the highest quality was obtained with the hybrid HBcAg-CRBDH6-H-PTFZn nanoparticle, which elicited a response twofold more potent -though not reaching statistical significance (p= 0.1562)- than the hybrid HBcAg-CRBDH6-H-PTFNi nanoparticle, which was the second most potent of the evaluated immunogens.

Hybrid HBcAg-CRBDH6-H-PTFZn nanoparticles induce an immune response over one order of magnitude more potent -63-fold- that the natural response detected in the SARS-CoV-2 convalescent serum panel (p=0.0049). The presence of a metal ion in hybrid nanoparticles is of paramount importance for the quality of the response against RBD, as omitting the ion in HBcAg-CRBDH6-H-PTF nanoparticle results in a response that is 3.9-fold less potent (P=0.0287, G5 vs G6).

In all cases the hybrid nanoparticles outperformed the chimeric protein: HBcAg-CRBDH6-H-PTFZn, HBcAg-CRBDH6-H-PTFNi and HBcAg-CRBDH6 were 19.5-, 9- and 5-fold more potent than CRBDH6-H (p= 0.0065, p= 0.0012, p=0.1699 respectively), although without a metal the difference is not statistically significant. This result is consistent with the importance of the quaternary and supramolecular structure underlying the design of the chimeric proteins disclosed in the present invention. On the other hand, as observed in the inset of Figure 22B, the response against the chimeric CRBDH6-H protein was three-fold higher compared with the titers observed in convalescent sera, although the difference, again, was not statistically significant (p=0.6862).

The sera from the different groups were also evaluated by an assay for neutralization of viral infection in cultured cells. For this purpose, two homogeneous mixtures were prepared by mixing equal volumes of 5 and 5 sera, respectively, from group G2 (negative control, HBcAg). In the case of groups G3 (CRBDH6-H-PTFNi) and G4 (HBcAg-CRBDH6-H-PTFNi), five different mixtures were prepared for each by pairing the sera with the closest anti-RBD IgG titers. Figure 22C shows the results of the analysis of these mixtures in the *in vitro* neutralization assay for SARS-CoV-2. As can be observed, the resulting data is consistent with that obtained for the ACE2/RBD binding inhibition assay. The means of the neutralizing titers were 0, 496 and 1280 for groups G2, G3 and G4, respectively, confirming the functional superiority of the antibodies generated by hybrid HBcAg-CRBDH6-H-PTFNi nanoparticles when compared with those generated by the chimeric protein CRBDH6-H alone.

Summarizing, the results show that the hybrid nanoparticles (which include metals) are 10-20-fold more immunogenic than protein CRBDH6 alone, and that the presence of metal ions is strictly required for this effect. Pertaining the quality of the response, the hybrid nanoparticles are up to 19-fold more potent than protein CRBDH6, and up to 80% of said response depends on the presence of metal ions. There was a correlation between biological data and the stability of hybrid nanoparticles, as the more stable nanoparticles were the most immunogenic with the most potent inhibition of receptor binding by RBD.

## Claims

1. A chimeric protein having a modular structure, comprising:
a) A receptor-binding domain (RBD) from the coronavirus Spike protein (S) that comprises a segment of said domain from protein S.
b) A segment able to bind the nucleocapsid antigen of the Hepatitis B Virus (HBcAg) that comprises the amino acid sequence WSFFSNI.
c) A segment that comprises the amino acid sequence HHHHHH.
d) A flexible 5-12 amino acids spacer segment formed by the amino acids G and S.
e) A 7-20 amino acids spacer segment that is selected between a segment that comprises SSSSSSSSSS and the segment STNLAAA,
wherein segments from (a) to (e) are arranged in (b)-(d)-(a)-(e)-(c) order or in (c)-(e)-(a)-(d)-(b) order.

2. The chimeric protein according to claim 1 wherein the RBD segment (a) is a segment that is located between amino acids Q320 and C590 of protein S from SARS-CoV-2.

3. The chimeric protein according to claim 1 wherein the flexible 5-12 amino acids spacer segment (d) has an amino acid sequence selected from the group composed of sequences GGSGG, GGSSGGS, GGGSSGGG, GGGSSGGSSGGG and GGSGGSGGS.

4. The chimeric protein according to claim 1 wherein the 7-20 amino acids spacer segment (e) has an amino acid sequence selected from the group composed of sequences GGSGGSSSSSSSSSSIE, SSGSSSSSSSSSS and GGSGGSSSSSSSSSSGGGIE.

5. The chimeric protein according to claim 1, having the amino acid sequence identified as SEQ ID NO: 4 or SEQ ID NO: 5.

6. A vaccine composition comprising the chimeric protein according to claim 1 and pharmaceutically acceptable excipients or diluents.

7. The vaccine composition according to claim 6 containing additionally a vaccine adjuvant.

8. The vaccine composition according to claim 7 wherein the vaccine adjuvant is an aluminum salt.

9. The vaccine composition according to claim 6 containing additionally the nucleocapsid antigen from the Hepatitis B Virus (HBcAg).

10. The vaccine composition according to claim 9 wherein the chimeric protein and HBcAg are forming hybrid nanoparticles.

11. The vaccine composition according to claim 6 containing additionally a second coronavirus antigen.

12. The vaccine composition according to claim 6 administered through the parenteral route, the mucosal route, or a combination thereof.

13. The vaccine composition according to claims 6 to 12 wherein the chimeric protein is selected from the amino acid sequences consisting of SEQ ID NO: 4 or SEQ ID NO: 5.

14. The use of the chimeric protein according to claim 1 to manufacture a vaccine composition for the prevention of an infection caused by a coronavirus.

15. The use of claim 14 wherein the infection is caused by SARS-CoV-2.

16. The use of claim 14 wherein the vaccine composition is administered through the parenteral route, the mucosal route, or a combination thereof in a simultaneous or sequential manner during the course of an immunization scheme.

17. The use according to claims 14 to 16 wherein the chimeric protein is selected from the amino acid sequences consisting of SEQ ID NO: 4 or SEQ ID NO: 5.

18. A method to prevent an infection caused by a coronavirus **characterized by** the administration to the affected individual of a pharmaceutically effective amount of a vaccine composition comprising the chimeric protein from claim 1 and pharmaceutically acceptable excipients or diluents.

19. The prevention method according to claim 18 wherein the vaccine composition contains additionally the nucleocapsid antigen from the Hepatitis B Virus (HBcAg).

20. The prevention method according to claim 18 wherein the vaccine composition is administered through the parenteral route, the mucosal route, or a combination thereof.
